(19) Europäisches Patentamt
European Patent Office
Office européen des brevets

(11) Veröffentlichungsnummer: **0 301 402 B1**

(12) # EUROPÄISCHE PATENTSCHRIFT

(45) Veröffentlichungstag der Patentschrift: **21.08.91 Patentblatt 91/34**

(51) Int. Cl.[5]: **C07C 323/00,** A61K 31/215, A61K 31/10, A61K 31/19, C07D 317/38, A61K 31/34

(21) Anmeldenummer: **88111747.7**

(22) Anmeldetag: **21.07.88**

(54) **Leukotrienantagonisten, Verfahren zu ihrer Herstellung und ihre Anwendung zur Behandlung von Krankheiten.**

(30) Priorität: **25.07.87 DE 3724669**

(43) Veröffentlichungstag der Anmeldung: **01.02.89 Patentblatt 89/05**

(45) Bekanntmachung des Hinweises auf die Patenterteilung: **21.08.91 Patentblatt 91/34**

(84) Benannte Vertragsstaaten: **AT BE CH DE ES FR GB GR IT LI LU NL SE**

(56) Entgegenhaltungen: **GB-A- 2 190 377**

(73) Patentinhaber: **HOECHST AKTIENGESELLSCHAFT**
**Postfach 80 03 20**
**W-6230 Frankfurt am Main 80 (DE)**

(72) Erfinder: **Wess, Günther, Dr.**
**Langenselbolder Weg 35**
**W-6455 Erlensee (DE)**
Erfinder: **Bartmann, Wilhelm, Prof. Dr.**
**Am Dachsbau 5**
**W-6232 Bad Soden am Taunus (DE)**
Erfinder: **Beck, Gerhard, Dr.**
**Gustav-Freytag-Strasse 24**
**W-6000 Frankfurt am Main (DE)**
Erfinder: **Anagnostopulos, Hiristo**
**Feldbergstrasse 6**
**W-6204 Taunusstein (DE)**

EP 0 301 402 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft neue chemische Verbindungen, die als solche oder in Form ihrer pharmakologisch verträglichen Salze leukotrienantagonistische Wirkung haben, Verfahren zur Herstellung dieser Verbindungen, pharmazeutische Mittel, welche die erfindungsgemäßen aktiven Verbindungen enthalten und deren Verwendung, insbesondere zur Behandlung von Krankheiten, die mit einem erhöhten Leukotrienspiegel verbunden sind, z.B. Asthma.

Als Antwort auf verschiedene, z.B. durch Allergene hervorgerufene Reize schütten basophile Zellen und Mastzellen einen als SRS-A (Slow Reacting Substance of Anaphylaxis) bezeichneten Mediator aus, der sowohl im Tierversuch als auch am Menschen eine außerordentlich starke bronchokonstriktorische Wirkung zeigt und vermutlich eine wichtige Rolle bei asthmatischen Erkrankungen spielt. Vor einigen Jahren konnte gezeigt werden, daß SRS-A ein Gemisch aus den Peptidoleukotrienen $LTC_4$, $LTD_4$ und $LTE_4$ darstellt, die aus Arachidonsäure über den sog. 5-Lipoxygenaseweg entstehen Es wird vermutet, daß die Leukotriene auch bei anderen allergischen und entzündlichen Erkrankungen, wie allergischen Hautreaktionen, Psoriasis, ulcerativer Colitis und rheumatischer Arthritis sowie beim Schock eine wichtige Rolle spielen.

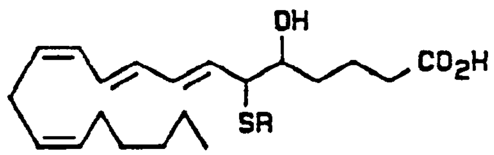

γ-Glu

$LTC_4$ R= Cys-Gly

$LTD_4$ R= Cys-Gly

$LTE_4$ R= Cys

Die biologische Wirkung der Leukotriene wird über spezifische Rezeptoren an den Zielzellen (glatte Muskelzellen, Makrophagen etc.) vermittelt. Deshalb sollten Verbindungen, die diese Rezeptoren blockieren können (d.h. Rezeptorantagonisten), zur Behandlung der oben angesprochenen Krankheiten geeignet sein.

Es ist bereits beschrieben worden, daß bestimmte Variationen an der Grundstruktur der Leukotriene (teilweise Sättigung der Doppelbindungen, Einbau eines Benzolringes in die Kette, Verkürzung, Abwandlung oder völliges Weglassen der Peptid-Seitenkette oder der endständigen Carboxylgruppe) zu partiellen Agonisten oder Antagonisten führen können (für eine Übersicht s. John H. Musser et al., Agents and Actions 18, 332-41 (1986), sowie John G. Gleason et. al., J. Med. Chem. 30(6), 959-61, (1987)). Viele der bisher beschriebenen Leukotrien-Analogen haben allerdings noch agonistische Eigenschaften oder sind von wenigen Ausnahmen abgesehen in vivo ungenügend bzw. nicht oral wirksam.

Wir haben nun überraschenderweise gefunden, daß man die Grundstruktur der Leukotriene noch erheblich stärker abwandeln kann, als bisher beschreiben, ohne die erwünschte antagonistische Wirkung zu verlieren.

Gegenstand der Erfindung sind daher neue Verbindungen der allgemeinen Formel I :

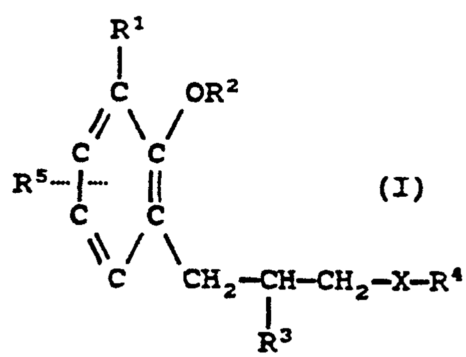

(I)

wobei die Reste folgende Bedeutung haben :

X ist O, S, SO oder $SO_2$ ;

$R^1$ ist H, $C_1$-$C_{12}$-Alkyl, $C_2$-$C_{12}$-Alkenyl oder $C_2$-$C_{12}$-Alkinyl (geradkettig oder verzweigt), $C_3$-$C_8$-Cycloalkyl, $C_3$-$C_8$-Cycloalkenyl, Halogen, Phenoxy, $CF_3$, $NO_2$, OH, $OR^6$, COOH, $COOR^6$, CHO oder $COR^7$ ;

$R_2$ ist H, $C_1$-$C_{12}$-Alkyl, $C_2$-$C_{12}$-Alkenyl oder $C_2$-$C_{12}$-Alkinyl (geradkettig oder verzweigt), $C_3$-$C_8$-Cycloalkyl, $C_3$-$C_8$-Cycloalkenyl, Phenyl-$C_1$-$C_{10}$-alkyl, Phenoxy-$C_1$-$C_{10}$-alkyl, Phenyl-$C_2$-$C_{10}$-alkenyl oder Phenyl-$C_2$-$C_{10}$-

alkinyl, wobei die Phenylringe noch durch 1-3 $C_1$-$C_4$-Alkyl, $C_2$-$C_4$-Alkenyl, $C_1$-$C_4$-Alkoxy, $C_1$-$C_4$-Alkanoyl, $C_1$-$C_4$-Alkoxycarbonyl, Hydroxy oder Halogenreste substituiert sein können, oder $R^2$ ist Pyridylmethyl oder Thienylmethyl ;

$R^3$ ist OH, $OR^6$, $OCOR^7$ ;

$R^4$ ist $C_1$-$C_4$-Alkyl, $C_2$-$C_4$-Hydroxyalkyl, $C_3$-$C_4$-Dihydroxyalkyl, $C_1$-$C_4$-Alkoxy-$C_1$-$C_4$-alkyl, $C_1$-$C_4$-Alkanoyloxy-$C_1$-$C_4$-alkyl, Phenyl oder Phenyl-$C_1$-$C_4$-alkyl, wobei die Phenylringe mit HO, Halogen, $C_1$-$C_4$-Alkoxy oder $C_1$-$C_4$-Alkylthio ein- oder zweifach substituiert sein können, oder $R^4$ ist eine Gruppe der allgemeinen Formel $(CH_2)_nCOR^8$ oder $(CH_2)_nR^9$ ;

$R^5$ ist H, Halogen, $CF_3$, OH, $C_1$-$C_4$-Alkyl oder $C_1$-$C_4$-Alkoxy ;

$R^6$ ist $C_1$-$C_4$-Alkyl, Allyl oder Benzyl ;

$R^7$ ist $C_1$-$C_4$-Alkyl oder Phenyl ;

$R^8$ ist OH, $C_1$-$C_4$-Alkoxy, $CH_2OH$, $OCH_2CO_2H$, $OCH_2CO_2R^7$, $OCH_2Ph$, NHOH, $NH_2$, $NHR^7$, $NR^7{}_2$, Piperidino, Pyrrolidino, Morpholino oder Phenoxy, wobei der Phenoxyrest noch durch Carboxy, $C_1$-$C_4$-Alkoxycarbonyl, OH oder $OCH_3$ substituiert sein kann ;

$R^9$ ist Tetrazol-5-yl oder 2-Oxo-1,3-dioxolan-4-yl ;

n ist 0, 1, 2 oder 3 ;

sowie physiologisch verträgliche Salze solcher Verbindungen der allgemeinen Formel I, in denen einer der Reste eine Carboxylgruppe (COOH) enthält.

Bevorzugt sind Verbindungen der allgemeinen Formel I, in der die Reste die folgende Bedeutung haben :

X ist O, S, SO oder $SO_2$ ;

$R^1$ ist H, $C_1$-$C_4$-Alkyl, $C_3$-$C_8$-Cycloalkyl oder $C_3$-$C_8$-Cycloalkenyl ;

$R^2$ ist H, $C_1$-$C_{10}$-Alkyl (geradkettig oder verzweigt), $C_3$-$C_8$-Cycloalkyl, Phenyl-$C_1$-$C_{10}$-alkyl oder Phenoxy-$C_1$-$C_{10}$-alkyl, wobei die Phenylringe noch durch 1-3 $C_1$-$C_4$-Alkyl, $C_2$-$C_4$-Alkenyl, $C_1$-$C_4$-Alkoxy, $C_1$-$C_4$-Alkanoyl, $C_1$-$C_4$-Alkoxycarbonyl, Hydroxy oder Halogenreste substituiert sein können, oder

$R^2$ ist Pyridylmethyl oder Thienylmethyl ;

$R^3$ ist OH ;

$R^4$ ist $C_1$-$C_4$-Alkyl, $C_2$-$C_4$-Hydroxyalkyl, $C_3$-$C_4$-Dihydroxyalkyl, $C_1$-$C_4$-Alkoxy-$C_1$-$C_4$-alkyl, $C_1$-$C_4$-Alkanoyloxy-$C_1$-$C_4$-alkyl, Phenyl oder Phenyl-$C_1$-$C_4$-alkyl, wobei die Phenylringe mit HO, Halogen, $C_1$-$C_4$-Alkoxy oder $C_1$-$C_4$-Alkylthio ein- oder zweifach substituiert sein können, oder $R^4$ ist eine Gruppe der allgemeinen Formel $(CH_2)_nCOR^8$ oder $(CH_2)_nR^9$ ;

$R^5$ ist H oder Halogen ;

$R^7$ ist $C_1$-$C_4$-Alkyl ;

$R^8$ ist OH, $C_1$-$C_4$-Alkoxy, $CH_2OH$, $OCH_2CO_2H$, $OCH_2CO_2R^7$, $OCH_2Ph$, NHOH, $NH_2$, $NHR^7$, $NR^7{}_2$, Piperidino, Pyrrolidino, Morpholino, oder Phenoxy, wobei der Phenoxyrest noch durch Carboxy, $C_1$-$C_4$-Alkoxycarbonyl, OH oder $OCH_3$ substituiert sein kann ;

$R^9$ ist Tetrazol-5-yl ;

n ist 1, 2 oder 3.

Besonders bevorzugt sind Verbindungen der allgemeinen Formel I, in der die Reste die folgende Bedeutung haben :

X is S ;

$R^1$ ist H, $CH_3$ oder Cyclopentyl ;

$R^2$ ist H, Benzyl, wobei der Benzylrest am Phenylring noch durch Methoxy oder Chlor ein- oder zweifach substituiert sein kann, Phenoxy-$C_2$-$C_4$-alkyl, wobei der Phenylring noch mit 1-3 $C_1$-$C_4$-Alkyl, $C_1$-$C_4$-Alkanoyl oder Hydroxygruppen substituiert sein kann, Pyridylmethyl oder Thienylmethyl ;

$R^3$ ist OH ;

$R^4$ ist $C_1$-$C_4$-Alkyl, $C_2$-$C_4$-Hydroxyalkyl, $C_3$-$C_4$-Dihydroxyalkyl, $C_1$-$C_4$-Alkoxy-$C_1$-$C_4$-alkyl, $C_1$-$C_4$-Alkanoyloxy-$C_1$-$C_4$-alkyl, Phenyl oder Benzyl, wobei die Phenylringe mit HO, Methoxy oder Methylthio ein- oder zweifach substituiert sein können, oder $R^4$ ist eine Gruppe der allgemeinen Formel $(CH_2)_nCOR^8$ oder $(CH_2)_nR^9$ ;

$R^5$ ist H oder Chlor ;

$R^7$ ist $C_1$-$C_4$-Alkyl ;

$R^8$ ist OH, $C_1$-$C_4$-Alkoxy, $CH_2OH$, $OCH_2CO_2H$, $OCH_2CO_2R^7$, $OCH_2Ph$, NHOH, $NH_2$, Piperidino, Pyrrolidino, Morpholino, oder Phenoxy, wobei der Phenoxyrest noch durch Carboxy, $C_1$-$C_4$-Alkoxycarbonyl, OH oder $OCH_3$ substituiert sein kann ;

$R^9$ ist Tetrazol-5-yl ;

n ist 1, 2 oder 3.

Insbesondere sind die folgenden Verbindungen bevorzugt :

(6RS)-7-(2-Benzyloxyphenyl)-6-hydroxy-4-thiaheptansäuremethylester

(6RS)-7-(2-Benzyloxy-3-cyclopentylphenyl)-6-hydroxy-4-thiaheptansäuremethylester

(6RS)-7-(2-Benzyloxy-3-methylphenyl)-6-hydroxy-4-thiaheptansäuremethylester
(6RS)-7-(2-Benzyloxy-3,5-dimethylphenyl)-6-hydroxy-4-thiaheptansäuremethylester
(6RS)-7-(2-Benzyloxy-3-cyclopentylphenyl)-6-hydroxy-4-thiaheptansäure
(6RS)-7-(2-Benzyloxy-5-chlorphenyl)-6-hydroxy-4-thiaheptansäuremethylester
(5RS)-6-(2-Benzyloxy-3-cyclopentylphenyl)-3-thiahexan-1,5-diol

Wie aus der allgemeinen Formel I ersichtlich ist, enthalten die erfindungsgemäßen Verbindungen ein asymmetrisches Kohlenstoffatom (jenes, an dem der Rest $R^3$ sitzt). Gegenstand der Erfindung sind daher nicht nur die Racemate der erfindungsgemäßen Verbindungen, sondern auch deren Enantiomeren.

Gegenstand der Erfindung sind weiterhin Verfahren zur Herstellung der erfindungsgemäßen Verbindungen der Formel I.

Das Verfahren zur Herstellung der Verbindungen der Formel I ist dadurch gekennzeichnet, daß man A) eine Verbindung der allgemeinen Formel II,

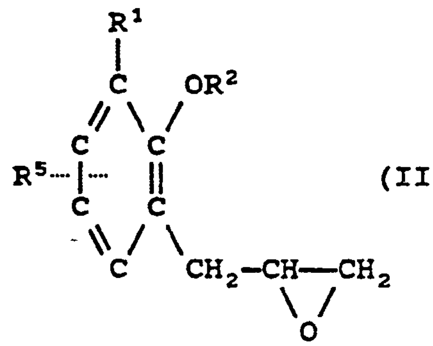

(II)

in der die Reste $R^1$, $R^2$ und $R^5$ die zu Formel I genannte Bedeutung haben, mit einer Verbindung der allgemeinen Formel $R^4XH$, wobei $R^4$ die zu Formel I genannte Bedeutung hat und X O oder S ist, zu einer Verbindung der Formel I umsetzt, worin $R^1$, $R^2$, $R^4$ und $R^5$ die angegebene Bedeutung haben, $R^3$ die Hydroxygruppe ist und X Schwefel oder Sauerstoff bedeutet, oder B) eine Verbindung der allgemeinen Formel III,

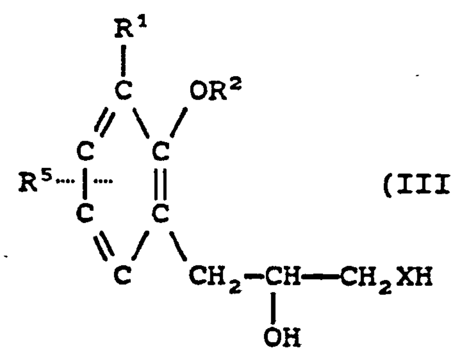

(III)

in der die Reste $R^1$, $R^2$ und $R^5$ die zu Formel I genannte Bedeutung haben, und X O oder S bedeutet, mit einer Verbindung der allgemeinen Formel $R^4Y$, wobei $R^4$ die zu Formel I genannte Bedeutung hat und Y eine Abgangsgruppe wie z.B. Cl, Br, J, $OSO_2CH_3$, $OSO_2Ph$, $OSO_2Tl$, $OSO_2CF_3$ oder $OSO_2R^4$ ist, zu einer Verbindung der Formel I umsetzt, worin $R^1$, $R^2$, $R^4$ und $R^5$ die angegebene Bedeutung haben, $R^3$ die Hydroxygruppe ist und X Schwefel oder Sauerstoff bedeutet, und gegebenenfalls eine erhaltene Verbindung durch Umwandlung in andere Verbindungen der Formel I überführt.

Die Herstellung der erfindungsgemäßen Verbindungen der Formel I, in denen der Rest $R^3$=OH ist kann auf den im

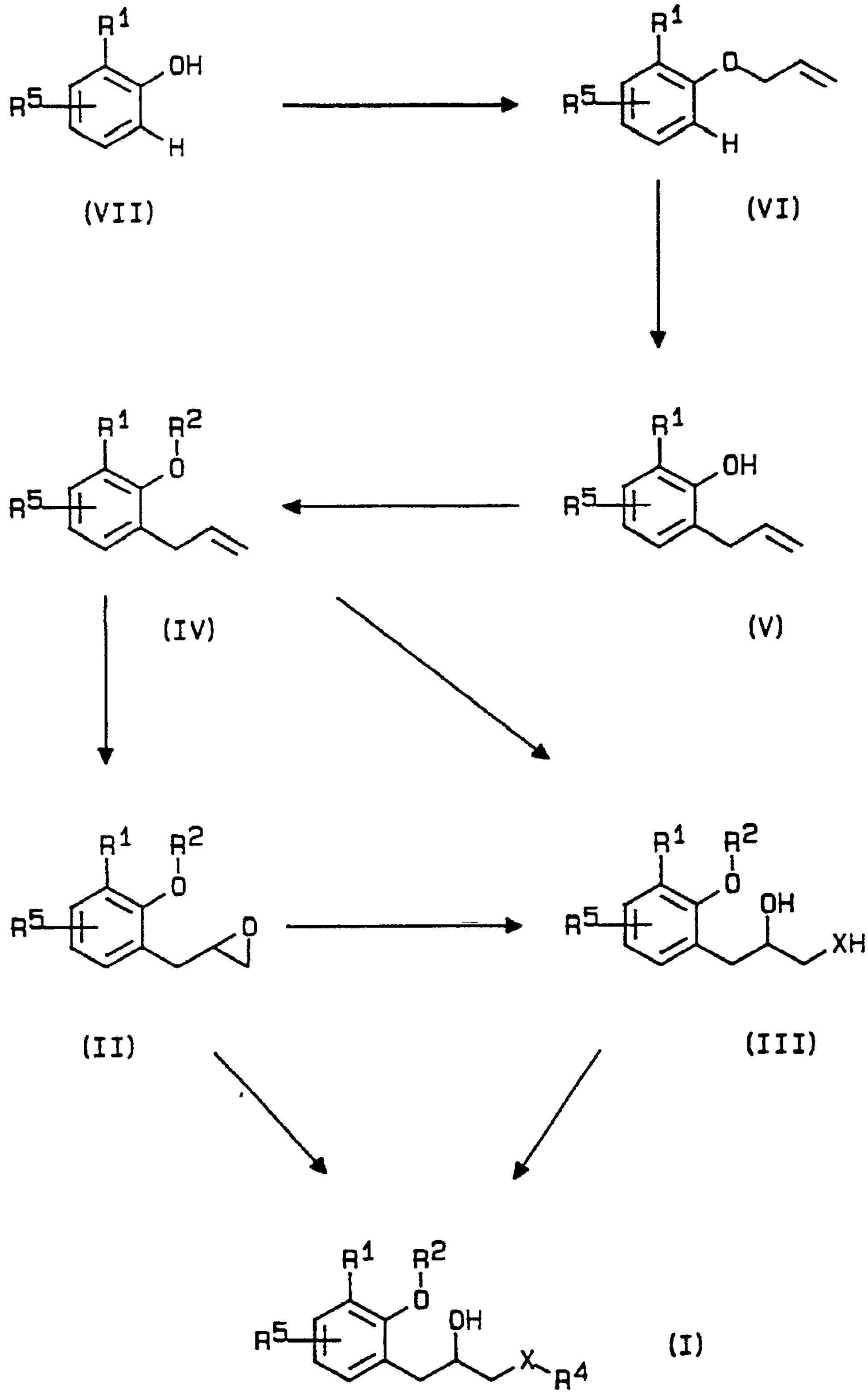

(VII) (VI) (V) (IV) (II) (III) (I)

Schema

Schema zusammengefaßten Wegen erfolgen.

**Verfahren A** ist dadurch charakterisiert, daß man ein Epoxyd der allgemeinen Formel II, in der $R^1$, $R^2$ und $R^5$ die gleiche Bedeutung haben, wie in Formel I, mit einem Alkohol der allgemeinen Formel $R^4OH$ oder einem Mercaptan der allgemeinen Formel $R^4SH$ umsetzt, wobei $R^4$ die für Formel I genannte Bedeutung hat. Die Umsetzung erfolgt vorteilhafterweise in einem inerten organischen Lösungsmittel, wie Toluol, Tetrahydrofuran, Diethylether, tert.Butylmethylether, Dimethylformamid oder dimethylsulfoxyd — für die Mercaptane sind auch niedere Alkohole wie Methanol, Ethanol, Isopropanol oder tert. Butanol geeignet — in Gegenwart einer Base. Als Basen sind im Fall der Alkohole $R^4OH$ besonders starke Basen wie Kalium-tert.butylat, Natriumhydrid oder Lithiumalkyle (bevorzugt n-Butyllithium) geeignet ; bevorzugt ist Natriumhydrid. Im Fall der Mercaptane $R^4SH$ können auch schwächere Basen wie tert. Amine, besonders Trialkylamine, bevorzugt Triethylamin, Diisopropylethylamin oder 1,4-Diazabicyclo[2,2,2]octan (DABCO), oder tertiäre Amidine, besonders bicyclische Amidine, bevorzugt 1,5-Diazabicyclo[4,3,0]non-5-en (DBN) oder 1,8-Diazabicyclo[5,4,0]undec-7-en (DBU), verwendet werden. Wenn man die Umsetzung mit den Mercaptanen $R^4SH$ in einem niederen Alkohol durchführt, können vorteilhaft auch die entsprechenden Natrium- oder Kaliumalkoholate als Base verwendet werden.

Die Reaktion wird bei Temperaturen von −20°C bis zum Siedepunkt des verwendeten Lösungsmittels durchgeführt. Bevorzugt ist ein Temperaturbereich von 0 bis 120°C, besonders von 20 bis 80°C.

Die bevorzugte Ausführungsform des Verfahrens A zur Herstellung von erfindungsgemäßen Verbindungen der Formel I, in denen $R^3$=OH und X=O ist, besteht in der Reaktion einer Verbindung der Formel II mit einem Alkohol $R^4OH$ in Gegenwart von Natriumhydrid in Dimethylformamid oder Tetrahydrofuran bei Temperaturen von 20 bis 60°C. Die Reaktion wird dabei vorteilhafterweise unter Feuchtigkeitsausschluß oder einem Schutzgas (Stickstoff oder Argon) durchgeführt.

Die bevorzugte Ausführungsform des Verfahrens A zur Herstellung von erfindungsgemäßen Verbindungen der Formel I, in denen $R^3$=OH und X=S ist, besteht in der Reaktion einer Verbindung der Formel II mit einem Mercaptan $R^4SH$ in Gegenwart von Triethylamin oder DBU in Tetrahydrofuran oder Methanol (falls die Gruppe $R^4$ einen Ester enthält, wird vorteilhafterweise der in diesem Ester enthaltene Alkohol verwendet) bei Temperaturen von 20 bis 60°C. Die Reaktion wird dabei vorteilhafterweise unter einem Schutzgas (Stickstoff oder Argon) durchgeführt.

Die für das Verfahren A notwendigen Ausgangsmaterialien der allgemeinen Formel II lassen sich nach dem Fachmann bekannten Standard-Epoxidierungsverfahren aus den entsprechenden Olefinen der allgemeinen Formel IV erhalten (s. Houben-Weyl, Methoden der organischen Chemie, Band VI/3, S. 385 ff). Diese wiederum sind aus den 2-Allylphenolen der allgemeinen Formel V leicht durch Alkylierung mit Halogeniden oder Sulfonaten des Typs $R^2Y$ nach Standardverfahren (s. Houben-Weyl, Band VI/3 S. 49 ff) herzustellen. 2-Allylphenole der allgemeinen Formel V lassen sich durch Claisen-Umlagerung von Allylphenylethern der allgemeinen Formel VI erhalten (s. Houben-Weyl, Band VI/1c S. 502 ff). Diese wiederum sind, soweit nicht kommerziell erhältlich, durch Alkylierung von Phenolen der allgemeinen Formel VII mit Allylchorid sehr einfach zugänglich. Für die Herstellung der Phenole VII, soweit sie nicht käuflich sind, steht eine große Zahl von Standardverfahren zur Verfügung (s. Houben-Weyl, Band VI 1c).

**Verfahren B** ist dadurch charakterisiert, daß man eine Verbindung der allgemeinen Formel III, in der $R^1$, $R^2$ und $R^5$ die gleiche Bedeutung haben, wie in Formel I und X O oder S ist, mit einer Verbindung der allgemeinen Formel $R^4Y$ umsetzt, wobei $R^4$ die für Formel I genannte Bedeutung hat und Y eine Abgangsgruppe wie Cl, Br, J oder $OSO_2Z$ (Z=$CH_3$, Ph, Tolyl, $CF_3$, $OR^4$) ist. Die Umsetzung erfolgt vorteilhafterweise in einem inerten organischen Lösungsmittel, wie Toluol, Tetrahydrofuran, Diethylether, tert.Butylmethylether, Dimethylformamid oder Dimethylsulfoxyd — für die Verbindung der allgemeinen Formel III, in denen X=S ist, sind auch niedere Alkohole wie Methanol, Ethanol, Isopropanol oder tert. Butanol geeignet — in Gegenwart einer Base. Als Basen sind im Fall der Verbindung der allgemeinen Formel III, in denen X=O ist, besonders starke Basen wie Kalium-tert.butylat, Natriumhydrid oder Lithiumalkyle (bevorzugt n-Butyllithium) geeignet ; bevorzugt ist Natriumhydrid. Im Fall der Verbindungen der allgemeinen Formel III, in denen X=S ist, können auch schwächere Basen wie tert. Amine, besonders Trialkylamine, bevorzugt Triethylamin, Diisopropylethylamin oder 1,4-Diazabicyclo[2,2,2]octan (DABCO), oder tertiäre Amidine, besonders bicyclische Amidine, bevorzugt 1,5-Diazabicyclo[4,3,0]non-5-en (DBN) oder 1,8-Diazabicyclo[5,4,0]undec-7-en (DBU), verwendet werden. Wenn man die Umsetzung mit den Verbindung der allgemeinen Formel III, in denen X=S ist, in einem niederen Alkohol durchführt, können vorteilhaft auch die entsprechenden Natrium- oder Kaliumalkoholate als Base verwendet werden.

Die Reaktion wird bei Temperaturen von −20°C bis zum Siedepunkt des verwendeten Lösungsmittels durchgeführt. Bevorzugt ist ein Temperaturbereich von 0 bis 120°C, besonders von 20 bis 80°C.

Verfahren B ist weniger geeignet zur Herstellung solcher Verbindungen der Formel I, in denen der Rest $R^4$ Phenyl ist.

Die bevorzugte Ausführungsform des Verfahrens B zur Herstellung von erfindungsgemäßen Verbindungen der Formel I, in denen $R^3$=OH und X=O ist, besteht in der Reaktion einer Verbindung der allgemeinen Formel III, in der X=O ist, mit einer Verbindung $R^4Y$ in Gegenwart von Natriumhydrid in Dimethylformamid oder Tetrahydrofuran bei Temperaturen von 20 bis 60°C. Die Reaktion wird dabei vorteilhafterweise unter Feuchtigkeitsausschluß oder einem Schutzgas (Stickstoff oder Argon) durchgeführt.

Die bevorzugte Ausführungsform des Verfahrens B zur Herstellung von erfindungsgemäßen Verbindungen der Formel I, in denen $R^3$=OH und X=S ist, besteht in der Reaktion einer Verbindung der Formel III, in der X=S ist, mit einer Verbindung $R^4Y$ in Gegenwart von Triethylamin, Natriummethanolat oder DBU in Tetrahydrofuran oder Methanol (falls die Gruppe $R^4$ einen Ester enthält, wird vorteilhafterweise der in diesem Ester enthaltene Alkohol verwendet) bei Temperaturen von 20 bis 60°C. Die Reaktion wird dabei vorteilhafterweise unter einem Schutzgas (Stickstoff oder Argon) durchgeführt.

Die für das Verfahren B benötigten Ausgangsverbindungen der allgemeinen Formel III können nach dem Fachmann geläufigen Verfahren (Houben-Weyl, Band VI/3, S. 454 ff (für X=O) und S. 461 ff (für X=S)) aus den Epoxyden der allgemeinen Formel II hergestellt werden. Besonders geeignet für die Herstellung der Verbindung der allgemeinen Formel III, in denen X=S ist, ist die bekannte Öffnung von Epoxyden mit Thioessigsäure mit anschließender alkalischer Hydrolyse des resultierenden Thioesters. Verbindungen der allgemeinen Formel III, in denen X=O ist, lassen sich auch aus den Olefinen der allgemeinen Formel IV durch Hydroxylierung nach Standardverfahren (Osmiumtetroxyd, Ameisensäure/$H_2O_2$ etc.) erhalten.

Eine Reihe von erfindungsgemäßen Verbindungen der allgemeinen Formel I lassen sich auch durch Umwandlung anderer erfindungsgemäßer Verbindungen der Formel I erhalten.

Verbindung der allgemeinen Formel I, in denen $R^3$=$OR^6$ ist, lassen sich aus Verbindung der allgemeinen Formel I, in der $R^3$=OH ist, durch Umsetzung mit einer Verbindung der allgemeinen Formel $R^6Y$ erhalten, wobei $R^6$ die für Formel I genannte Bedeutung hat und Y eine Abgangsgruppe wie Cl, Br, J oder $OSO_2Z$ (Z=$CH_3$, Ph, Tolyl, $CF_3$, $OR^6$) ist. Die Umsetzung erfolgt vorteilhafterweise in einem inerten organischen Lösungsmittel, wie Toluol, Tetrahydrofuran, Diethylether, tert.Butylmethylether, Dimethylformamid oder Dimethylsulfoxyd in Gegenwart einer Base. Als Basen sind besonders starke Basen wie Kalium-tert.butylat, Natriumhydrid oder Lithiumalkyle (bevorzugt n-Butyllithium) geeignet ; bevorzugt ist Natriumhydrid.

Die Reaktion wird bei Temperturen von −20°C bis zum Siedepunkt des verwendeten Lösungsmittels durchgeführt. Bevorzugt ist ein Temperaturbereich von 0 bis 120°C, besonders von 20 bis 80°C.

Die bevorzugte Ausführungsform des Verfahrens zur Herstellung von erfindungsgemäßen Verbindungen der Formel I, in denen $R^3$=$OR^6$ ist, besteht in der Reaktion einer Verbindung der allgemeinen Formel I, in der $R^3$=OH ist, mit einer Verbindung $R^6Y$ in Gegenwart von Natriumhydrid in Dimethylformamid oder Tetrahydrofuran bei Temperturen von 20 bis 70°C. Die Reaktion wird dabei vorteilhafterweise unter Feuchtigkeitsausschluß oder einem Schutzgas (Stickstoff oder Argon) durchgeführt.

Verbindung der allgemeinen Formel I, in denen $R^3$=$OCOR^7$ ist, lassen sich aus Verbindung der allgemeinen Formel I, in der $R^3$=OH ist, durch Acylierung mit einer Verbindung der allgemeinen Formel $R^7COCl$, $R^7COBr$ oder $(R^7CO)_2O$ erhalten, wobei $R^7$ die für Formel I genannte Bedeutung hat. Die Umsetzung erfolgt vorteilhafterweise in Pyridin oder in einem Gemisch von Pyridin mit einem inerten organischen Lösungsmittel, wie Toluol, Tetrahydrofuran, Diethylether, Methylenchlorid oder tert.Butylmethylether. Die Reaktion kann durch Zugabe von 5-100 Mol% eines Acylierungskatalysators wie 4-Dimethylaminopyridin, 4-Pyrrolidinopyridin oder 4-Piperidinopyridin beschleunigt werden.

Die Reaktion wird bei Temperaturen von −20°C bis zum Siedepunkt des verwendeten Lösungsmittels durchgeführt. Bevorzugt ist ein Temperaturbereich von −20 bis 50°C, besonders von 0 bis 25°C.

Die bevorzugte Ausführungsform des Verfahrens zur Herstellung von erfindungsgemäßen Verbindungen der Formel I, in denen $R^3$=$OCOR^7$ ist, besteht in der Reaktion einer Verbindung der allgemeinen Formel I, in der $R^3$=OH ist, mit einem organischen Säurechlorid $R^7COCl$ oder -anhydrid $(R^7CO)_2O$ in Pyridin in Gegenwart von 10-20 Mol% 4-Dimethylaminopyridin bei Temperaturen von 0 bis 20°C. Die Reaktion wird dabei vorteilhafterweise unter Feuchtigkeitsausschluß oder einem Schutzgas (Stickstoff oder Argon) durchgeführt.

Verbindung der allgemeinen Formel I, in denen $R^4$ $C_1$-$C_4$-Alkanoyloxy-$C_2$-$C_4$-alkyl ist, lassen sich aus Verbindung der allgemeinen Formel I, in denen $R^4$ $C_2$-$C_4$-Hydroxyalkyl ist, durch Acylierung mit einem $C_1$-$C_4$-Carbonsäurechlorid oder -anhydrid erhalten. Die Umsetzung erfolgt vorteilhafterweise in Pyridin oder in einem Gemisch von Pyridin mit einem inerten organischen Lösungsmittel, wie Toluol, Tetrahydrofuran, Diethylether, Methylenchlorid oder tert.Butylmethylether. Die Reaktion kann durch Zugabe von 5-100 Mol% eines Acylierungskatalysators wie 4-Dimethylaminopyridin, 4-Pyrrolidinopyridin oder 4-Piperidinopyridin beschleunigt werden.

Die Reaktion wird bei Temperaturen von −20°C bis zum Siedepunkt des verwendeten Lösungsmittels durchgeführt. Bevorzugt ist ein Temperaturbereich von −20 bis 50°C, besonders von 0 bis 25°C.

Verbindung der allgemeinen Formel I, in denen $R^4$ $C_1$-$C_4$-Alkoxy-$C_2$-$C_4$-alkyl ist, lassen sich aus Verbindung der allgemeinen Formel I, in denen $R^4$ $C_2$-$C_4$-Hydroxyalkyl ist, durch Alkylierung mit einem $C_1$-$C_4$-Alkylhalogenid, -sulfonat oder -sulfat erhalten. Die Umsetzung erfolgt vorteilhafterweise in einem inerten organischen Lösungsmittel, wie Toluol, Tetrahydrofuran, Diethylether, tert.Butylmethylether, Dimethylformamid oder Dimethylsulfoxyd in Gegenwart einer Base. Als Basen sind besonders starke Basen wie Kalium-tert.butylat, Natriumhydrid oder Lithiumalkyle (bevorzugt n-Butyllithium) geeignet, bevorzugt ist Natriumhydrid.

Die Reaktion wird bei Temperaturen von −20°C bis zum Siedepunkt des verwendeten Lösungsmittels durchgeführt. Bevorzugt ist ein Temperaturbereich von 0 bis 120°C, besonders von 20 bis 80°C.

Verbindung der allgemeinen Formel I, in denen $R^4$ $(CH_2)_nCOOH$ und n 1-3 ist, lassen sich aus Verbindung der allgemeinen Formel I, in denen $R^4$ $C_2$-$C_4$-Hydroxyalkyl ist, durch Oxidation mit geeigneten Oxidationsmitteln erhalten, die dem Fachmann bekannt sind. Die Wahl des Oxidationsmittels wird durch die Art der sonstigen Reste im Molekül bestimmt. Geeignet sind z.B. Pyridiniumdichromat in Dimethylformamid, Chromschwefelsäure in Wasser, Essigsäure oder Aceton (Jones-Oxidation) oder Rutheniumtrichlorid (katalytische Mengen) in Gegenwart von Kooxidantien wie $K_2S_2O_8$ oder $NaJO_4$ in Wasser/$CCl_4$/Acetonitril oder Wasser/$CH_2Cl_2$-Systemen.

Die Reaktion wird bei Temperaturen von −20°C bis zum Siedepunkt des verwendeten Lösungsmittels durchgeführt. Bevorzugt ist ein Temperaturbereich von 0 bis 50°C, besonders von 0 bis 30°C.

Verbindung der allgemeinen Formel I, in denen $R^4$ $C_2$-$C_4$-Hydroxyalkyl ist, lassen sich aus Verbindung der allgemeinen Formel I, in denen $R^4$ $(CH_2)_nCOR^8$, n 1-3 und $R^8$ OH, $C_1$-$C_4$-Alkoxy, Phenoxy oder Benzyloxy ist, durch Reduktion mit geeigneten Reduktionsmitteln, die dem Fachmann bekannt sind, erhalten. Geeignet sind insbesondere komplexe Hydride, wie Boran (bevorzugt als Komplex mit Dimethylsulfid oder Tetrahydrofuran), Natriumborhydrid, Lithiumborhydrid, Lithiumaluminiumhydrid, Diisobutylaluminiumhydrid, Aluminiumhydrid. Die Umsetzung erfolgt vorteilhafterweise in einem inerten organischen Lösungsmittel, wie Toluol, Tetrahydrofuran, Diethylether, tert.-Butylmethylether oder Methylenchlorid.

Die Reaktion wird bei Temperaturen von −20°C bis zum Siedepunkt des verwendeten Lösungsmittels durchgeführt. Bevorzugt ist ein Temperaturbereich von 0 bis 100°C, besonders von 10 bis 70°C.

Verbindung der allgemeinen Formel I, in denen $R^4$ $C_2$-$C_4$-Hydroxyalkyl ist, lassen sich aus Verbindung der allgemeinen Formel I, in denen $R^4$ $C_1$-$C_4$-Alkanoyloxy-$C_2$-$C_4$-alkyl ist, durch Hydrolyse oder Aminolyse erhalten. Geignete Reagentien sind Basen wie Natriumhydroxyd, Kaliumhydroxyd, Bariumhydroxyd, Calciumhydroxyd, Kaliumcarbonat, Natriumcarbonat,Natrium- bzw. Kaliumalkoholate, Ammoniak, $C_1$-$C_4$-Alkylamine, Ethylendiamin oder 2-Aminoethanol in niederen Alkoholen oder Alkohol/Wasser-Gemischen als Lösungsmittel. Im Fall der Amine kann auch ohne Lösungsmittel gearbeitet werden.

Die Reaktion wird bei Temperaturen von −20°C bis zum Siedepunkt des verwendeten Lösungsmittels durchgeführt. Bevorzugt ist ein Temperaturbereich von 0 bis 100°C, besonders von 20 bis 70°C.

Verbindung der allgemeinen Formel I, in denen $R^4$ $(CH_2)_nCOR^8$ und $R^8$ OH ist, lassen sich aus Verbindung der allgemeinen Formel I, in denen $R^4$ $(CH_2)_nCOR^8$ und $R^8$ $C_1$-$C_4$-Alkoxy, Phenoxy oder Benzyloxy ist, durch Verseifung nach dem Fachmann bekannten Standardverfahren erhalten. Besonders geeignet ist die alkalische Verseifung mit Basen wie Natriumhydroxyd, Kaliumhydroxyd, Bariumhydroxyd, Calciumhydroxyd, Kaliumcarbonat oder Natriumcarbonat in einem niederen Alkohol oder Alkohol/ Wasser-Gemischen.

Die Reaktion wird bei Temperaturen von 0°C bis zum Siedepunkt des verwendeten Lösungsmittels durchgeführt. Bevorzugt ist ein Temperaturbereich von 20 bis 100°C, besonders von 20 bis 70°C.

Verbindung der allgemeinen Formel I, in denen $R^4$ $(CH_2)_nCOR^8$ und $R^8$ $C_1$-$C_4$-Alkoxy oder Benzyloxy ist, lassen sich aus Verbindung der allgemeinen Formel I, in denen $R^4$ $(CH_2)_nCOR^8$ und $R^8$ OH ist, durch Veresterung nach dem Fachmann bekannten Standardverfahren erhalten. Besonders geeignet ist die alkylierende Veresterung durch Reaktion mit einem $C_1$-$C_4$-alkylhalogenid, -sulfonat oder -sulfat bzw. Benzylchlorid oder -bromid in Gegenwart einer Base wie Kaliumcarbonat, Kaliumfluorid, 1,4-Diazabicyclo[2,2,2]octan (DABCO), 1,5-Diazabicyclo[4,3,0]non-5-en (DBN) oder 1,8-Diazabicyclo[5,4,0]undec-7-en (DBU) in einem polaren, aprotischen Lösungsmittel wie Aceton, Butanon, Acetonitril, N,N-Dimethylformamid, N,N-dimethylacetamid oder N-Methylpyrrolidon. Für die Herstellung des Methylesters ist auch die Reaktion mit Diazomethan in Diethylether, Tetrahydrofuran oder tert-Butylmethylether geeignet.

Die Reaktion wird bei Temperaturen von 0°C bis zum Siedepunkt des verwendeten Lösungsmittels durchgeführt. Bevorzugt ist ein Temperaturbereich von 20 bis 100°C, besonders von 20 bis 50°C.

Verbindung der allgemeinen Formel I, in denen $R^4$ $(CH_2)_nCOR^8$ und $R^8$ $C_1$-$C_4$-Alkoxy oder Benzyloxy ist, lassen sich auch aus anderen Verbindung der gleichen Formel durch Umesterung (Austausch des Alkoxy- bzw. Benzyloxyrestes durch einen anderen Alkoxyrest bzw. durch Benzyloxy) nach dem Fachmann bekannten Standardverfahren erhalten. Diese Umsetzung führt man bevorzugt in dem Alkohol als Lösungsmittel durch, der dem einzuführenden Rest entspricht ; als Katalysatoren können entweder Säuren wie Schwefelsäure, Chlorwasserstoff, p-Toluolsulfonsäure, Benzolsulfonsäure, Camphersulfonsäure, Methansulfonsäure oder saure Ionenaustauscherharze oder Basen wie Kaliumcarbonat, Natriumcarbonat, das dem als Lösungsmittel verwendeten Alkohol entsprechende Lithium-, Natrium-, Kalium- oder Titanalkoholat oder Titantetraisopropylat verwendet werden. Basische Katalysatoren sind in diesem Fall bevorzugt.

Die Reaktion wird bei Temperaturen von 0°C bis zum Siedepunkt des verwendeten Lösungsmittels durchgeführt. Bevorzugt ist ein Temperaturbereich von 20 bis 100°C, besonders von 50 bis 80°C.

Verbindung der allgemeinen Formel I, in denen $R^4$ $(CH_2)_nCOR^8$ und $R^8$ NHOH, $NH_2$, $NHR^7$, $NR^7_2$, Piperidino, Pyrrolidino oder Morpholino ist, lassen sich aus Verbindung der allgemeinen Formel I, in denen $R^4$ $(CH_2)_nCOR^8$ und $R^8$ OH ist, durch Kondensation mit dem entsprechenden Amin $R^8H$ nach Verfahren herstellen, die dem Fachmann im Prinzip bekannt sind. Geeignete Kondensationsmittel sind z.B. Carbonyldiimidazol, Dicyclohexylcarbodiimid, Diethoxyphosphonsäurechlorid, Diethoxyphosphonsäureazid, Phosphoroxychlorid, Propylphosphonsäureanhydrid und Diphenylphosphonsäurechlorid. Vorteilhafterweise führt man die Kondensation in einem Lösungsmittel durch. Geeignet sind, abhängig vom verwendeten Kondensationsmittel, nahezu alle gängigen organischen Lösungsmittel wie Kohlenwasserstoffe (gesättigt oder aromatisch), chlorierte Kohlenwasserstoffe, Ether, niedere Ketone wie Aceton oder Butanon, tert. Amide wie Dimethylformamid, Dimethylacetamid oder N-Methylpyrrolidon, niedere Akohole wie Methanol, Ethanol, Isopropanol, n-, iso- oder tert.Butanol und sogar wässrige Systeme bzw. Mischungen (homogen oder zweiphasig) der angeführten organischen Lösungsmittel mit Wasser.

Eine bevorzugte Ausführungsform dieses Verfahrens besteht in der Umsetzung der Verbindungen der allgemeinen Formel I, in denen $R^4$ $(CH_2)_nCOR^8$ und $R^8$ OH ist, mit Carbonyldiimidazol in einem aprotischen Lösungsmittel, besonders Tetrahydrofuran, bei Temperaturen von 0 bis 20°C, gefolgt von der Zugabe der Aminkomponente $R^8H$.

Alternativ kann man die Carbonsäurekomponente zunächst in ein aktiviertes Derivat (Säurechlorid, gemischtes Anhydrid) überführen und dieses dann mit dem Amin $R^8H$ umsetzen, vorzugsweise in Gegenwart einer Hilfsbase wie Natriumhydrogencarbonat, Natriumcarbonat, Kaliumcarbonat, Natronlauge, Kalilauge, oder einem tertiären Amin wie Pyridin, Lutidin oder einem Trialkylamin wie Triethylamin, Diisopropylethylamin oder Tributylamin. Für die Aktivierung von Carbonsäuren sind dem Fachmann eine große Zahl von Methoden geläufig, z.B. die Umsetzung mit Thionylchlorid, Phosphortrichlorid, Phosgen oder Oxalylchlorid zum Säurechlorid oder die Umsetzung mit Chlorameisensäureestern oder Sulfonsäurechloriden (Methansulfonsäurechlorid, Trifluormethansulfonsäurechlorid, Benzolsulfonsäurechlorid) in Gegenwart von Basen, vorzugsweise von tert. Aminen wie Triethylamin oder Pyridin, zu den gemischten Anhydriden.

Eine bevorzugte Ausführungsform dieses Verfahrens besteht in der Umsetzung der Verbindungen der allgemeinen Formel I, in denen $R^4$ $(CH_2)_nCOR^8$ und $R^8$ OH ist, mit Chlorameisensäureethylester in Gegenwart von Triethylamin in Methylenchlorid bei Temperaturen von –20 bis 5°C, gefolgt von der Zugabe der Aminkomponente $R^8H$.

Verbindung der allgemeinen Formel I, in denen $R^4$ $(CH_2)_nCOR^8$ und $R^8$ NHOH, $NH_2$, $NHR^7$, $NR^7_2$, Piperidino, Pyrrolidino oder Morpholino ist, lassen sich auch aus Verbindung der allgemeinen Formel I, in denen $R^4$ $(CH_2)_nCOR^8$ und $R^8$ $C_1$-$C_4$-Alkoxy, Benzyloxy oder Phenoxy ist, durch Aminolyse mit dem entsprechenden Amin $R^8H$ herstellen. Die Reaktion wird vorzugsweise in einem geeigneten Lösungsmittel wie einem Alkohol (Methanol, Ethanol, n-Propanol, n-Butanol, Isopropanol, 2-Ethoxyethanol, 2-Methoxyethanol), einem Ether (bevorzugt Tetrahydrofuran, Dioxan, 1,2-Dimethoxyethan, oder Diethylenglykoldimethylether) oder einem Kohlenwasserstoff wie Toluol, Xylol, Mesitylen, Tetralin oder Dekalin durchgeführt. Man kann auch einen Überschuß des Amins $R^8H$ als Lösungsmittel verwenden. Die Reaktion wird bei Temperaturen im Bereich von 20°C bis zum Siedepunkt des verwendeten Lösungsmittels durchgeführt, bevorzugt sind Temperaturen von 40 bis 120°C, besonders von 40 bis 80°C.

Besonders bei den niedrigsiedenden Aminen ist es vorteilhaft, die Reaktion unter Inertgasdruck ($N_2$ oder Argon 20-50 bar) im Autoklaven durchzuführen.

Es kann in einigen Fällen, besonders bei den niedrigsiedenden Aminen und im Fall des Hydroxylamins, vorteilhaft sein, anstelle des freien Amins ein Salz des Amins mit einer organischen oder anorganischen Säure einzusetzen und daraus im Reaktionsgemisch mit einer Hilfsbase das Amin freizusetzen. Geeignete Salze sind besonders die Hydrochloride, Hydrobromide, Hydrogensulfate, Sulfate oder Acetate ; geeignete Hilfsbasen sind Alkali- und Erdalkalicarbonate und -hydrogencarbonate, wie Natriumcarbonat, Natriumhydrogencarbonat, Kaliumcarbonat, Kaliumhydrogencarbonat, Calciumcarbonat, oder Alkalisalze organischer Säuren, wie Natriumacetat oder Kaliumacetat, oder tertiäre Amine, besonders Trialkylamine wie Triethylamin, Diisoproplyethylamin, Tributylamin oder Trioctylamin. Die Reaktion wird bevorzugt in einem Alkohol, z.B. Methanol, Ethanol, n-Propanol, n-Butanol, Isopropanol, 2-Ethoxyethanol oder 2-Methoxyethanol als Lösungsmittel bei Temperaturen von 20 bis 120°C, besonders von 40 bis 100°C durchgeführt, eventuell im Autoklaven unter Inertgasdruck.

Verbindungen der allgemeinen Formel I, in denen X SO oder $SO_2$ ist, lassen sich aus solchen Verbindungen der allgemeinen Formel I, in denen X S ist, durch Oxidation erhalten. Geeignete Oxidationsmittel sind vor allem organische Persäuren wie 3-Chlorperbenzoesäure, Peressigsäure, Perphthalsäure und Perbenzoesäure. Ebenfalls geeignet sind einige anorganische Oxidationsmittel wie Natriumperjodat, Kaliumpermanganat, Natriumhypochlorid, Natriumchlorid und Wasserstoffperoxyd.

Art und Menge des zu wählenden Oxidationsmittels hängen davon ab, ob man zur Stufe des Sulfoxyds oder des Sulfons oxidieren möchte. Für die selektive Oxidation zum Sulfoxyd sind besonders geeignet die oben aufgezählten organische Persäuren (1 Äquivalent, Reaktionstemperatur –40 bis 0°C) oder Natriumperjodat ; zur Oxidation bis zum Sulfon kann man entweder einen Überschuß (2-3 Äquivalente) organischer Persäure (Reaktionstemperatur 0 bis 40°C) verwenden oder eines der aufgeführten anorganischen Oxidationsmittel mit Ausnahme von Natriumperjodat.

Die Reaktionen mit Persäuren führt man bevorzugt in einem organische Lösungsmittel wie Methylenchlorid, Methanol, Ethanol, Toluol oder Tetrahydrofuran durch ; mit Wasserstoffperoxyd wird bevorzugt in Essigsäure oder Acetanhydrid umgesetzt ; für die Reaktion mit den anderen Oxidationsmitteln eignen sich besonders wässrige Systeme wie Mischungen von Methanol, Aceton oder Essigsäure mit Wasser.

Bei einigen der vorstehend geschilderten Verfahren kann es vorteilhaft sein, eine im Ausgangsmaterial vorhandene reaktive Gruppe, insbesondere eine Hydroxylgruppe, die nicht an der Reaktion teilnehmen soll, mit einer geeigneten Schutzgruppe zu blockieren. Bevorzugt sind Schutzgruppen, insbesondere Ether oder Carbonate, die sich unter milden, sauren oder neutralen Bedingungen oder hydrogenolytisch abspalten lassen, wie tert-Butyl-, Benzyl-, 4-Methoxybenzyl-, Benzhydryl-, Methoxymethyl-, 1-Ethoxyethyl oder Tetrahydropyranylether, Silylether wie Trimethylsilyl- oder tert.-Butyl-dimethylsilyl oder Carbonate wie Benzyloxycarbonyl-und tert.-Butoxycarbonylderivate, die aus der Peptid- und Steroidchemie wohlbekannt sind.

Diese Schutzgruppen können nach erfolgter Hauptreaktion in allgemein bekannter Weise, z.B. durch Behandeln mit organischen Säuren, wie Ameisensäure, Essigsäure, Trifluoressigsäure oder Oxalsäure oder einem Gemisch davon, optional in Gegenwart von Wasser und/oder inerten organischen Lösungsmitteln, wie niederen Alkoholen (z.B. Methanol oder Ethanol) oder cyclischen Ethern (z.B. Tetrahydrofuran oder Dioxan) unter Freisetzung des Hydroxyls entfernt werden. Für die Abspaltung von Silylschutzgruppen sind Fluoride wie KF, CsF oder $Bu_4NF$ geeignet. Für die Abspaltung von Benzyl, Benzhydryl, 4-Methoxybenzyl oder Benzyloxycarbonyl-Schutzgruppen ist auch die Hydrierung in Gegenwart eines geeigneten Katalysators, z.B. Palladium, Platin, Platinoxyd oder Nickel, geeignet. Diese Reaktion erfolgt bevorzugt in einem organischen Lösungsmittel, insbesondere in einem niederen Alkohol wie Methanol oder Ethanol oder in Essigsäure, eventuell mit Zusatz von Wasser, bei Wasserstoffdrücken von 1 bis 200 bar, bevorzugt von 1 bis 100 bar, bei Temperaturen von 20 bis 100°C, bevorzugt bei 20 bis 60°C, insbesondere bei Raumtemperatur (20-30°C).

Die Trennung der bei der Synthese anfallenden Racemate der erfindungsgemäßen Verbindungen der allgemeinen Formel I in die beiden Enantiomeren, die ebenfalls Gegenstand der Erfindung sind, erfolgt bevorzugt durch chromatographische Trennung an einem optisch aktiven Trägermaterial. Geeignete Materialien sind z.B. Triacetylcellulose, Tribenzoylcellulose oder mit Dinitrobenzoyl-phenylglycin modifiziertes Kieselgel (sog. Perkle-Phasen).

Für die Racematspaltung der erfindungsgemäßen Verbindungen der allgemeinen Formel I, in denen $R^3$=OH ist, ist auch eine Derivatisierung dieser OH-Gruppe mit einer optisch aktiven Carbonsäure (als Ester) oder einem optisch aktiven Isocyanat (als Carbamat), anschließende chromatographische Trennung der resultierenden Diastereomeren und schließlich Rückspaltung des Derivats geeignet. Als optisch aktive Hilfsstoffe sind besonders geeignet Isocyanate wie Dehydroabietylisocyanat oder (R) bzw. (S)-1-(1- Naphthyl)ethylisocyanat oder N-geschützte natürliche Aminosäuren, wie (S)-N-Methansulfonylphenylalanin. Die Derivatisierung und die Rückspaltung erfolgen nach dem Fachmann geläufigen Standardverfahren.

Die erfindungsgemäßen Verbindungen der allgemeinen Formel I können, soweit sie eine Carboxylgruppe enthalten, Salze mit anorganischen oder organischen Basen bilden. Daher sind auch solche Salze Gegenstand der vorliegenden Erfindung. Bevorzugt sind Salze mit anorganischen Basen, besonders die physiologisch unbe-denklichen Alkalimetall-Salze, vor allem Natrium- und Kaliumsalze.

Die erfindungsgemäßen Verbindungen der allgemeinen Formel I haben wertvolle pharmakologische Eigenschaften ; insbesondere antagonisieren sie die Wirkung der Leukotriene. Zur Charakterisierung der pharmakologischen Eigenschaften wurden die folgenden experimentellen Modelle verwendet :

Zur Bestimmung der Leukotrien-antagonistischen Wirkung der erfindungsgemäßen Substanzen der allgemeinen Formel I dient die Inhibierung der durch Leukotriene hervorgerufenen Kontraktion von Meerschweinchen-Lungenstreifen. Die verwendete Methode ist eine Modifikation des von Foreman, Shelly und Weber (Arch. Int. Pharamcodyn. 278, 193-206 (1985)) beschriebenen Tests.

Meerschweinchen werden durch eine Überdosis Ether getötet. Die Brusthöhle wird geöffnet ; die Lungen werden entfernt und in Streifen von 5 cm Länge geschnitten, die in physiologischer Kochsalzlösung aufbewahrt werden. Zur Messung werden die Lungenstreifen in ein mit Ringer-Lösung gefülltes Organbad gebracht, das mit Carbogen ($O_2/CO_2$ 95 : 5 Volumenteile) begast wird und auf 37 °C temperiert ist. Man läßt die Streifen unter einer Last von 0.5-1 g für 30-60 Minuten äquilibrieren. Vor Beginn des Versuchs werden die Lungenstreifen

mit Indomethacin ($10^{-6}$ g/ml Badflüssigkeit) vorbehandelt.

Die Kontraktion wird durch $LTC_4$, $LTD_4$ oder $LTE_4$-Gabe in einer Konzentration von 3 ng/ml Badflüssigkeit hervorgerufen. Die Testsubstanzen werden therapeutisch, nach Erreichen des maximalen Kontraktionsplateaus, in mehreren Konzentrationen und in Zeitabständen von 10 Minuten ins Bad appliziert. Pro Konzentration der Testsubstanz werden 6-12 Lungenstreifen verwendet.

Angegeben sind die Konzentrationen der Testsubstanzen, bei denen die Kontraktion um 50% vermindert wird ($IC_{50}$) in µg/ml.

Nachstehend werden beispielhaft die Ergebnisse des pharmakologischen Tests für die folgenden Verbindungen aufgeführt:

| | |
|---|---|
| Verbindung A: | (6RS)-7-Benzyloxyphenyl)-6-hydroxy-4-thiaheptansäuremethylester |
| Verbindung B: | (6RS)-7-(2-Benzyloxy-3-cyclopentylphenyl)-6-hydroxy-4-thiaheptansäuremethylester |
| Verbindung C: | (6RS)-7-(2-Benzyloxy-3-cyclopentylphenyl)-6-hydroxy-4-thiaheptansäure |
| Verbindung D: | (6RS)-7-(2-Benzyloxy-5-chlorphenyl)-6-hydroxy-4-thiaheptansäuremethylester |
| Verbindung E: | (5RS)-6-(2-Benzyloxy-3-cylopentylphenyl)-3-thiahexan-1,5-diol |

| | $IC_{50}$ [µg/ml] gegenüber | | |
|---|---|---|---|
| Verbindung | $LTC_4$ | $LTD_4$ | $LTE_4$ |
| A | 0.6-1.0 | 0.6 | 0.6-1.0 |
| B | 0.6 | 1.0 | 3.0 |
| C | 3.0-6.0 | 3.0-6.0 | 3.0 |
| D | 6.0-10 | 3.0 | 1.0-3.0 |
| E | 6.0-10 | 6.0-10 | 1.0-3.0 |

Die erfindungsgemäßen Verbindungen sind aufgrund ihrer pharmakologischen Eigenschaften für die Behandlung allergischen und entzündlichen Krankheiten wie Asthma, allergischen Hautreaktionen, Psoriasis, ulcerativer Colitis oder rheumatischer Arthritis sowie Schock geeignet.

Die Erfindung betrifft daher weiter die Anwendung der erfindungsgemäßen Verbindungen der Formel I bei der Behandlung und Prophylaxe der vorstehend aufgeführten Krankheiten.

Die Erfindung umfaßt weiterhin die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere erfindungsgemäße Verbindung der allgemeinen Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche, oder vorzugsweise in Kombination mit geeigneten pharmazeutischen Hilfsstoffen in Form von Tabletten, Dragees, Kapseln, Suppositiorien, Emulsionen, Suspensionen, Cremes, Salben, Granulaten, Pulvern oder Lösungen eingesetzt, wobei der Wirkstoffgehalt vorteilhafterweise zwischen 0,1 und 95 % beträgt.

Welche Hilfsstoffe für die gewünschte Arzneimittelformulierung geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können topisch, oral, parenteral, intravenös, rektal oder durch Inhalation appliziert werden, wobei die bevorzugte Applikationsart von der zu behandelnden Krankheit abhängig ist.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Dareichungsformen gebracht, wie Tabletten, Dragees, Steckkapseln, wässrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke verwendet werden. Dabei kann die Zubereitung sowohl als Trocken- als auch als Feuchtgranulat

erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen oder deren physiologisch verträglichen Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage Wasser, physiologische Kochsalzlösung oder Alkohole, z.B. Ethanol, Propanol, Glycerin, daneben auch Zuckerlösungen wie Glucose- oder Mannitlösungen, oder auch eine Mischung aus den verschiedenen genannten Lösungsmitteln.

Pharmazeutische Präparate für topische und lokale Verwendung sind z.B. für die Behandlung der Haut Lotionen und Cremes, die eine flüssige oder semifeste Öl-in-Wasser- oder Wasser-in-Öl-Emulsion enthalten, und Salben (wobei solche verzugsweise ein Konservierungsmittel enthalten). Für die Behandlung der Augen eignen sich Augentropfen, welche die aktive Verbindung in wässriger oder öliger Lösung enthalten. Für die Behandlung der Nase sind Aerosole und Sprays, ähnlich den weiter unten beschriebenen für die Behandlung der Atemwege, grobe Puder, die durch schnelles Inhalieren durch die Nasenlöcher verabreicht werden, und vor allem Nasentropfen, welche die aktive Verbindungen in wässriger oder öliger Lösung enthalten, geeignet.

Als pharmazeutische Formulierungen für die Verabreichung in Form von Aerosolen oder Sprays sind geeignet z.B. Lösungen, Suspensionen oder Emulsionen des erfindungsgemäßen Wirkstoffs der allgemeinen Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0.1 bis 10, insbesondere von etwa 0.3 bis 3 Gewischts-%.

Die Dosierung des zu verabreichenden Wirkstoffs der Formel I und die Häufigkeit der Verabreichung hängen von der Wirkstärke und Wirkdauer der verwendeten Verbindung ab ; außerdem auch von Art und Stärke der zu behandelnden Krankheit sowie von Geschlecht, Alter, Gewicht und individueller Ansprechbarkeit des zu behandelnden Säugers. Im Durchschnitt dürfte die empfohlene tägliche Dosis einer erfindungsgemäßen Verbindung der Formel I bei einem etwa 75 kg schweren Säuger — in erster Linie einem Menschen — im Bereich von etwa 10 bis 500 mg, vorzugsweise zwischen etwa 25 und 250 mg liegen, wobei die Verabreichung nach Bedarf in mehreren Dosen pro Tag erfolgen kann.

Die folgenden Beispiele sollen die vorliegende Erfindung illustrieren, ohne sie jedoch in ihrem Umfang einzuschränken.

Rf-Werte wurden auf Kieselgel-Fertigplatten (5 × 10 cm, Schichtdicke 0.25 mm, Kieselgel 60 $F_{256}$) der Fa. Riedel de Haen bestimmt. Angegebene Laufmittelverhältnisse sind Volumenverhältnisse. Bei NMR-Spektren sind angegeben : Meßfrequenz in MHz, Lösungsmittel, für jedes Signal chemische Verschiebung in ppm (relativ zu Tetramethylsilan als Standard), Multiplizität, ev. Kopplungskonstanten in Hz und Zahl der Protonen laut Integration. Multiplizitäten werden durch folgende Abkürzungen charakterisiert : s = Singulett, d = Dublett, t = Triplett, q = Quartett, m = Multiplett, AB = AB-System ; kompliziertere werden durch Kombination dieser Buchstaben gekennzeichnet, z.B. dt = Dublett von Tripletts ; breite Signale sind durch den Zusatz "br" gekennzeichnet.

Massenspektren wurden entweder durch Elektronenstoß-Ionisation, Fast Atom Bombardment (FAB) oder Direkter Chemischer Ionisation (DCI) aufgenommen.

Der Fortschritt der Reaktionen wurde i. A. durch Dünnschicht-Chromatographie verfolgt ; Reaktionszeiten sind daher nur beispielhaft angegeben. Das Einengen von Lösungen erfolgte mittels eines Rotationsverdampfers bei einem Druck von 1-200 Torr und Badtemperaturen von 20-80°C, je nach Lösungsmittel.

Sofern kein Schmelzpunkt angegeben ist, handelt es sich bei der betreffenden Verbindung um eine Flüssigkeit.

<u>Beispiel 1</u>

7-(2-Benzyloxy-3-cyclopentylphenyl)-6-hydroxy-4-thiaheptansäuremethylester

a) 486,7 g (3,0 Mol) 2-Cyclopentylphenol, 472 g (3,9 Mol) Allylbromid und 830 g (6,0 Mol) Kaliumcarbonat werden in 2,5 l Aceton unter gutem Rühren 26 h unter Rückfluß erhitzt. Die abgekühlte Reaktionsmischung wird filtriert und das Filtrat im Vakuum eingeengt. Der Rückstand wird mit Petrolether aufgenommen und dreimal mit 2n Natronlauge gewaschen. Die organische Phase wird über Magnesiumsulfat getrocknet, im Vakuum eingeengt und der Rüchstand schließlich im Vakuum destilliert. Man erhält 593 g (97% d. Theorie) (2-Cyclopentylphenyl)allylether.
Kp. 134°C/12 Torr Rf = 0,65 (Cyclohexan/ Essigester 5 : 1)

$C_{14}H_{18}O$ : Massenspektrum (MS) Molpeak : m/e = 202
$^1$H-NMR (60 MHz, $CDCl_3$, δ ppm) :
1.4-2.2 (m, 8H), 3.35 (m, 1H), 4.25 (m, 2H), 5.0-5.35, 5.45, 5.7-6.4 (jeweils m, Σ 3H), 6.6-7.2 (m, 4H)

Analog werden hergestellt :
(4-Chlorphenyl)allylether $C_9H_9ClO$ MS m/e = 168
(2,4-Dimethylphenyl)allylether $C_{11}H_{14}O$ MS m/e = 162

b) 290 g (1.43 Mol) (2-Cyclopentylphenyl)allylether werden unter Stickstoffatmosphäre 4h auf 220°C erhitzt. Das Rohprodukt wird durch Säulenchromatographie auf Kieselgel (Laufmittel Cyclohexan/ Essigester 50 : 1) gereinigt. Man erhält neben 20 g 4-Allyl-2-cyclopentylphenol 203 g (70% d. Th.) 2-Allyl-6-cyclopentylphenol.
Rf = 0.42 (0.19 für das p-Isomer) Cyclohexan/Essigester 9 : 1
$C_{14}H_{18}O$ MS m/e = 202
$^1$H-NMR (60 MHz, $CDCl_3$ δ ppm) :
1.4-2.2 (m,8H), 3.0-3.5 (m,3H), 5.0, 5.2, 5.6-6.4 (jeweils m, Σ 3H), 6.6-7.2 (m, 3H)

Analog wurden hergestellt :
2-Allyl-4-chlorphenol $C_9H_9ClO$ MS m/e = 168
2-Allyl-4,6-dimethylphenol $C_{11}H_{14}O$ MS m/e = 162

c) 90 g (0.44 Mol) 2-Allyl-6-cyclopentylphenol, 62 g (0.49 Mol) Benzylchlorid und 124 g (0.9 Mol) Kaliumcarbonat werden in 800 ml Aceton 70 h unter Rückfluß erhitzt. Die abgekühlte Reaktionsmischung wird filtriert und das Filtrat eingeengt. Der Rückstand wird in Petrolether aufgenommen, dreimal mit 2n Natronlauge und dann je einmal mit Wasser und gesättigter Kochsalzlösung gewaschen. Die organische Phase wird über Natriumsulfat getrocket und im Vakuum eingeengt. Der Rückstand wird im Vakuum destillert. Man erhält 118 g (92% d. Th.) 1-Allyl-2-benzyloxy-3-cyclopentyl-benzol.
Kp. 165-170°C/ 0.1-0.5 Torr $C_{21}H_{24}O$ MS m/e = 292
Rf = 0.58 (LM Cyclohexan/Essigester 9 : 1)
$^1$H-NMR (60 MHz, $CDCl_3$, δ ppm) :
1.4-2.2 (m,8H), 3.4 (m,30// 4.75 (s, 2H), 4.83, 5.1, 5.6-6.2 (jeweils m, Σ 3H), 6.8-7.4 (m,8H)

Analog wurden hergestellt :
1-Allyl-2-benzyloxybenzol $C_{16}H_{16}O$ MS m/e = 224
1-Allyl-2-benzyloxy-5-chlorbenzol $C_{16}H_{15}ClO$ MS m/e = 258
1-Allyl-2-benzyloxy-3,5-dimethylbenzol $C_{18}H_{20}O$ MS m/e = 252
1-Allyl-2-benzyloxy-3-methylbenzol $C_{17}H_{18}O$ MS m/e = 238
1-Allyl-2-(3-methoxybenzyl)-3-cyclopentylbenzol $C_{22}H_{26}O_2$ MS m/e = 322
1-Allyl-2-(2-methoxybenzyl)-3-cyclopentylbenzol $C_{22}H_{26}O_2$ MS m/e = 322
1-Allyl-2-(4-methoxybenzyl)-3-cyclopentylbenzol $C_{22}H_{26}O_2$ MS m/e = 322
1-Allyl-2-[3-(4-acetyl-3-hydroxy-2-propylphenoxy)propyloxy]-3-cyclopentylbenzol $C_{32}H_{44}O_7S$ MS m/e = 572

d) 20 g (0.0684 Mol) 1-Allyl-2-benzyloxy-3-cyclopentylbenzol werden in 200 ml Dichlormethan gelöst. Unter Rühren und Kühlung im Eisbad gibt man portionsweise 14 g (0.081 Mol) m-Chlorperbenzoesäure zu. Man läßt auf Raumtemperatur kommen und gibt nach 2.5 h nochmals 5 g m-Chlorperbenzoesäure zu. Man rührt über Nacht bei Raumtemperatur weiter, filtriert dann von der ausgefallenen m-Chlorbenzoesäure ab und engt das Filtrat im Vakuum ein. Der ölige Rückstand wird gut mit n-Pentan verrührt, filtriert und das Filtrat dreimal mit 2n Natronlauge und dann einmal mit gesättigter Kochsalzlösung gewaschen. Nach dem Trocknen über Natriumsulfat destilliert man das Lösungsmittel im Vakuum ab. Man erhält 23.9 g (2-Benzyloxy-3-cyclopentylbenzyl)oxiran als Öl.
$C_{21}H_{24}O_2$ MS m/e = 308
$^1$H-NMR (60 MHz, $CDCl_3$, δ ppm) :
1.4-2.2 (m, 8H), 2.2-3.8 (m, Σ 4H), 4.8 (s, 2H), 7.0-7.6 (m, 8H)

Analog wurden hergestellt
(2-Benzyloxybenzyl)oxiran $C_{16}H_{16}O_2$ MS m/e = 240
(2-Benzyloxy-5-chlorbenzyl)oxiran $C_{16}H_{15}ClO_2$ MS m/e = 274
(2-Benzyloxy-3,5-dimethylbenzyl)oxiran $C_{18}H_{20}O_2$ MS m/e = 268
(2-Benzyloxy-3-methylbenzyl)oxiran $C_{17}H_{18}O_2$ MS m/e = 254
[3-Cyclopentyl-2-(3-methoxybenzyloxy)benzyl]oxiran $C_{22}H_{26}O_3$ MS m/e = 338
[3-Cyclopentyl-2-(2-methoxybenzyloxy)benzyl]oxiran $C_{22}H_{26}O_3$ MS m/e = 338
[3-Cyclopentyl-2-(4-methoxybenzyloxy)benzyl]oxiran $C_{22}H_{26}O_3$ MS m/e = 338
{2-[3-(4-acetyl-3-hydroxy-2-propylphenoxy)propyloxy]-3-cyclopentylbenzyl}oxiran $C_{32}H_{44}O_8S$ MS m/e = 588

e) 20 g (59 mMol) (2-Benzyloxy-3-cyclopentylbenzyl)oxiran, 24.5 g (0.204 Mol) 3-Mercaptopropionsäuremethylester und 26.1 g (0.257 Mol) Triethylamin werden in 150 ml trockenem Methanol über Nacht bei Raumtemperatur unter Schutzgas (Argon oder Stickstoff) ge-rührt. Das Lösungsmittel wird im Vakuum abdestilliert und der Rückstand über eine Keiselgelsäule chromatographiert (Gradient Dichlormethan → Essigester). Man erhält 19.0 g (65% d. Th.) 7-(2-Benzyloxy-3-cyclopentylphenyl)-6-hydroxy-4-thiaheptansäuremethylester.
Rf = 0.34 (Cyclohexan/Essigester 2 : 1)
$C_{25}H_{32}O_4S$ MS m/e = 428
$^1$H-NMR (270 MHz, $CDCl_3$, δ ppm) :
1.5-1.9 und 2.1 (m, Σ 8H), 2.55 (t, 2H), 2.65 (m, 2H), 2.79 (m, 2H), 2.90 (dd, 2H), 3.41 (m, 1H), 3.68 (s, 3H), 4.00 (m, 1H), 4.87 (s, 2H), 7.09, 7.20, 7.3-7.5 (jeweils m, Σ 8H)

Analog zu Beispiel 1 wurden die folgenden Verbindungen hergestellt :

Beispiel 2

7-(2-Benzyloxy-5-chlorphenyl)-6-hydroxy-4-thiaheptansäuremethylester

$C_{20}H_{23}ClO_4S$ MS m/e = 394
$^1$H-NMR (60 MHz, $CDCl_3$, δ in ppm) :
2.6-3.0 (m, 9H), 3.72 (s, 3H), 3.8-4.2 (m, 1H), 5.07 (s, 2H), 6.6-7.2 (m, 3H), 7.40 (s, 5H)

Beispiel 3

7-(2-Benzyloxy-3,5-dimethylphenyl)-6-hydroxy-4-thiaheptansäuremethylester

$C_{22}H_{28}O_4S$ MS m/e = 388
Rf = 0.23 (Cyclohexan/Essigester 4 : 1)
$^1$ H-NMR (60 MHz, $CDCl_3$, δ in ppm) :
2.30 (s, 6H), 2.4-3.0 (m, 9H), 3.70 (s, 3H), 4.0 (m, 1H), 4.85 (s, 2H), 6.93 (s br, 2H), 7.44 (s br, 5H)

Beispiel 4

7-(2-Benzyloxy-3-methylphenyl)-6-hydroxy-4-thiaheptansäuremethylester

$C_{21}H_{26}O_4S$ MS m/e = 374
Rf = 0.21 (Cyclohexan/Essigester 4 : 1)
$^1$H-NMR (60 MHz, $CDCl_3$, δ in ppm) :
2.35 (s, 3H), 2.4-3.0 (m, 9H), 3.70 (s, 3H), 4.0 (m, 1H), 4.87 (s, 2H), 7.10 (s br, 3H), 7.45 (s br, 5H)

Beispiel 5

7-(2-Benzyloxyphenyl)-6-hydroxy-4-thiaheptansäuremethylester

$C_{20}H_{24}O_4S$ MS m/e = 360
Rf = 0.20 (Cyclohexan/Essigester 4 : 1)
$^1$H-NMR (60 MHz, $CDCl_3$, δ in ppm) :
2.4-3.0 (m, 9H), 3.70 (s, 3H), 3.8-4.2 (m, 1H), 5.12 (s, 2H), 6.8-7.5 (m) + 7.43 (s br) Σ 9H

Beispiel 6

7-[3-Cyclopentyl-2-(3-methoxybenzyloxy)phenyl]-6-hydroxy-4-thiaheptansäuremethylester

$C_{28}H_{34}O_5S$ MS m/e = 458
Rf = 0.15 (Cyclohexan/Essigester 4 : 1)
$^1$ H-NMR (60 MHz, $CDCl_3$, δ in ppm) :
1.3-2.2 (m, 8H), 2.4-3.0 (m, 9H), 3.3-3.6 (m, 1H), 3.70 (s, 3H), 3.87 (s, 3H), 3.8-4.2 (m, 1H), 4.86 (s, 2H), 6.8-7.5 (m, 7H)

Beispiel 7

7-[3-Cyclopentyl-2-(2-methoxybenzyloxy)phenyl]-6-hydroxy-4-thiaheptansäuremethylester

$C_{28}H_{34}O_5S$ MS m/e = 458
Rf = 0.14 (Cyclohexan/Essigester 4 : 1)
$^1$ H-NMR (60 MHz, $CDCl_3$), δ in ppm) :
1.3-2.2 (m, 8H), 2.4-3.0 (m, 9H), 3.3-3.6 (m, 1H), 3.70 (s, 3H), 3.87 (s, 3H), 3.8-4.2 (m, 1H), 4.86 (s, 2H), 6.8-7.6 (m, 7H)

Beispiel 8

7-[3-Cyclopentyl-2-(4-methoxybenzyloxy)phenyl]-6-hydroxy-4-thiaheptansäuremethylester

$C_{28}H_{34}O_5S$ MS m/e = 458
Rf = 0.12 (Cyclohexan/Essigester 4 : 1)
$^1$H-NMR (60 MHz, $CDCl_3$, δ in ppm) :
1.3-2.2 (m, 8H), 2.4-3.0 (m, 9H), 3.3-3.6 (m, 1H), 3.70 (s, 3H), 3.86 (s, 3H), 3.8-4.2 (m, 1H), 4.80 (s, 2H), 6.97 (d, 8-9Hz) + 6.8-7.5 (m) + 7.43 (d, 8-9Hz) Σ 7H

Beispiel 9

6-(2-Benzyloxy-3-cyclopentylphenyl)-5-hydroxy-3-thiahexansäuremethylester

$C_{24}H_{30}O_4S$ MS m/e = 414
Rf = 0.18 (Cyclohexan/Essigester 4 : 1)
$^1$H-NMR (60 MHz, $CDCl_3$, δ in ppm) :
1.3-2.2 (m, 8H), 2.5-3.1 (m, 5H), 3.25 (s, 2H), 3.3-3.6 (m, 1H), 3.70 (s, 3H), 3.8-4.2 (m, 1H), 4.86 (s, 2H), 7.0-7.3 (m, 3H), 7.43 (s br, 5H)

Beispiel 10

6-(2-Benzyloxy-3-cyclopentylphenyl)-3-thiahexan-1,5-diol

$C_{23}H_{30}O_3S$ MS m/e = 386
$^1$H-NMR (60 MHz, $CDCl_3$), δ in ppm) :
1.2-2.2 (m, 8H), 2.5-3.0 (m, 6-7H), 3.70 (t, 6Hz, 2H), 4.03 (quintuplett br, 5-6 Hz, 1H), 4.88 (s, 2H), 7.0-7.3 (m, 3H), 7.45 (s br, 5H)

Beispiel 11

7-(2-Benzyloxy-3-cyclopentylphenyl)-4-thiaheptan-1,2,6-triol

$C_{24}H_{32}O_4S$ MS m/e = 416
$^1$H-NMR (270 MHz, $CDCl_3$, δ in ppm) :
1.5-1.75 (m, 4H), 1.75-1.9 (m, 2H), 2.0-2.1 (m, 2H), 2.5-2.6 (m, 2H), 2.65-2.75 (m, 2H), 2.87 (m, 2H), 3.417 (quintuplett, 9Hz, 1H), 3.492 (dd, 11Hz, 7Hz, 1H), 3.6-3.8 (m, 2H), 4.03 (m, 1H), 5.866 (s, 2H), 7.052 (dd, 7-8Hz, 3Hz, 1H), 7.104 (t, 7-8Hz, 1H), 7.242 (dd, 7-8Hz, 3Hz, 1H), 7.3-7.5 (m, 5H)

Beispiel 12

7-{2-[3-(4-acetyl-3-hydroxy-2-propylphenoxy)propyloxy]-3-cyclopentylphenyl}-6-hydroxy-4-thiaheptansäuremethylester

$C_{32}H_{44}O_7S$ MS m/e = 572
$^1$H-NMR (270 MHz, $CDCl_3$, δ in ppm) :
0.925 (t, 8Hz, 3H), 1.45-1.6 (m, 8), 1.7-1.8 (m, 2H), 1.9-2.05 (m, 2H), 2.326 (quintuplett, 6Hz, 2H), 2.478 (dd, 14Hz, 8Hz, 1H) ; 2.581 (s, 3H), 2.55-2.7 (m, 6H), 2.75-2.85 (m, 4H), 3.259 (quintuplett, 8-9Hz, 1H), 3.693 (s,

3H), 3.9-4.1 (m, 3H), 4.326 (t, 6Hz, 2H), 6.518 (d, 8Hz, 1H), 7.0-7.1 (m, 2H), 7.13-7.2 (m, 1H), 7.625 (d, 8Hz, 1H), 12.75 (s, 1H)

Beispiel 13

6-[3-Cyclopentyl-2-(3-methoxybenzyloxy)phenyl]-5-hydroxy-3-thiahexansäuremethylester

$C_{25}H_{32}O_5S$ MS m/e = 444
$^1$H-NMR (60 MHz, $CDCl_3$, δ in ppm) :
1.3-2.2 (m, 8H), 2.4-3.1 (m, 5H), 3.26 (s, 2H), 3.3-3.6 (m, 1H), 3.70 (s, 3H), 3.87 (s, 3H), 3.8-4.2 (m, 1H), 4.86 (s, 2H), 6.8-7.5 (m, 7H)

Beispiel 14

7-(2-Benzyloxy-3-cyclopentylphenyl)-6-hydroxy-4-thiaheptansäure

0.675 g (1.63 mMol) 7-(2-Benzyloxy-3-cyclopentylphenyl)-6-hydroxy-4-thiaheptansäuremethylester (Beispiel 1) werden in 10 ml Methanol/Wasser 9 : 1 gelöst und mit 0.7 g (17.5 mMol) Na OH in wenig Wasser gelöst versetzt. Man läßt über Nacht bei Raumtemperatur rühren, destilliert dann das Lösungsmittel im Vakuum ab und säuert den Rückstand mit kalter 1n Salzsäure an. Man extrahiert dreimal mit Diethylether, wäscht die organischen Phasen zweimal mit ges. Kochsalzlösung, trocknet sie über Natriumsulfat und destilliert das Lösungsmittel im Vakuum ab. Man erhält 444 mg (66% d. Th.)
7-(2-Benzyloxy-3-cyclopentylphenyl)-6-hydroxy-4-thiaheptansäure.
Rf = 0.46 (LM Chloroform/Methanol 4 : 1)
$C_{24}H_{30}O_4S$ MS m/e = 414
$^1$H-NMR (60 MHz, $CDCl_3$, δ ppm) :
1.5-2.2 (m), 2.4-3.0 (m), 3.50 (m, 1H), 4.00 (m, 1H), 4.85 (s, 2H), 7.0-7.6 (m, 8H)

Analog zu Beispiel 14 wurden hergestellt :

Beispiel 15

7-(2-Benzyloxy-5-chlorphenyl)-6-hydroxy-4-thiaheptansäure

$C_{19}H_{21}ClO_4S$ MS m/e = 380
$^1$ H-NMR (60 MHz, $CDCl_3$, δ in ppm) :
2.6-3.0 (m, 9H), 3.8-4.2 (m, 1H), 4.99 (s, 2H), 6.6-7.2 (m, 4H), 7.32 (s, 5H)

Beispiel 16

7-(2-Benzyloxy-3,5-dimethylphenyl)-6-hydroxy-4-thiaheptansäure

$C_{21}H_{26}O_4S$ MS m/e = 374

Beispiel 17

7-(2-Benzyloxy-3-methylphenyl)-6-hydroxy-4-thiaheptansäure

$C_{20}H_{24}O_4S$ MS m/e = 360

Beispiel 18

7-(3-Cyclopentyl-2-(3-methoxybenzyloxy)phenyl]-6-hydroxy-4-thiaheptansäure

$C_{25}H_{32}O_5S$ MS m/e = 444

Beispiel 19

6-(2-Benzyloxy-3-cyclopentylphenyl)-5-hydroxy-3-thiahexansäure

$C_{23}H_{28}O_4S$ MS m/e = 400

Beispiel 20

6-[3-Cyclopentyl-2-(3-methoxybenzyloxy)phenyl]-5-hydroxy-3-thiahexansäure

$C_{24}H_{30}O_5S$ MS m/e = 430

Beispiel 21

7-(2-Benzyloxy-3-cyclopentylphenyl)-6-hydroxy-3-thiaheptansäure Natriumsalz

Variante 1 :

100 mg 7-(2-Benzyloxy-3-cyclopentylphenyl)-6-hydroxy-4-thiaheptansäure (Beispiel 10) werden in 5 ml Methanol gelöst und mit 10 mg Natriumhydroxyd in 0.5 ml Wasser versetzt. Man läßt 1 h bei Raumtemperatur rühren und dampft dann das Lösungsmittel in Vakuum ab. Der Rückstand wird mit Toluol versetzt und im Vakuum eingedampft (zweimal wiederholen). Der Rückstand wird an der Ölpumpe getrocknet. Man erhält 100 mg des Produkts.
Rf = 0.35 (Chloroform/Methanol 5 : 1)

Variante 2 :

0.1 g 7-(2-Benzyloxy-3-cyclopentylphenyl)-6-hydroxy-4-thiaheptansäuremethylester (Beispiel 1) werden in wenig Methanol/Wasser 9 : 1 gelöst und mit 1.1 Äquivalenten 2n Natronlauge versetzt. Man läßt über Nacht bei Raumtemperatur rühren, destilliert dann das Lösungsmittel in Vakuum ab und arbeitet weiter wie unter Variante 1 beschrieben auf. Man erhält 85 mg des Produkts.
Rf = 0.35 (Chloroform/Methanol 5 : 1)

Beispiel 22

(+)-7-(2-Benzyloxy-3-cyclopentylphenyl)-6-hydroxy-4-thiaheptansäuremethylester und

Beispiel 23

(−)-7-(2-Benzyloxy-3-cyclopentylphenyl)-6-hydroxy-4-thiaheptansäuremethylester

Racemischer 7-(2-Benzyloxy-3-cyclopentylphenyl)-6-hydroxy-4-thiaheptansäuremethylester (Beispiel 1) wird durch HPLC an Acetylcellulose (Laufmittel Ethanol) in die Enantiomeren getrennt. Die spektroskopischen Daten sind mit denen des Racemats identisch.

Beispiel 24

7-(2-Benzyloxy-3-cyclopentylphenyl)-6-hydroxy-4-thiaheptan-1,6-diol

1 g (2.33 mMol) 7-(2-Benzyloxy-3-cyclopentylphenyl)-6-hydroxy-4-thiaheptansäuremethylester (Beispiel 1) werden in 3 ml Tetrahydrofuran gelöst und zu 250 mg Lithiumaluminiumhydrid in 20 ml Tetrahydrofuran getropft. Man läßt 30 Min. bei Raumtemperatur rühren und gibt dann vorsichtig nacheinander 0.25 ml Wasser, 0.25 ml 15%-ige Natronlauge und 0.73 ml Wasser zu. Man filtriert den Niederschlag ab, wäscht das Filtrat einmal mit gesättigter Kochsalzlösung, trocknet über Magnesiumsulfat und destilliert das Lösungsmittel im Vakuum ab. Der Rückstand wird durch Chromatographie an Kieselgel gereinigt (Laufmittel Cyclohexan/Essigester 1 : 1). Man erhält 745 mg 7-(2-Benzyloxy-3-cyclopentylphenyl)-6-hydroxy-4-thiaheptan-1,6-diol
$C_{24}H_{32}O_3S$ MS m/e = 400
$^1$H-NMR (60 MHz, $CDCl_3$, $\delta$ ppm) :

1.4-2.2 (m, 10-11H), 2.4-3.0 (m, 6-7H), 3.2-4.2 (m) + 3.71 (t, 6Hz) Σ 4H), 4.90 (s, 2H), 7.0-7.3 (m, 3H), 7.45 (s br, 5H)

Beispiel 25

7-(2-Benzyloxy-3-cyclopentylphenyl)-6-hydroxy-4-thiaheptansäureethylester

1 g (2.33 mMol) 7-(2-Benzyloxy-3-cyclopentylphenyl)-6-hydroxy-4-thiaheptansäuremethylester (Beispiel 1) werden in 5 ml Ethanol gelöst und mit 2.5 g Kaliumcarbonat versetzt. Man läßt über Nacht unter Feuchtigkeitsausschluß rühren, filtriert und dampft das Filtrat im Vakuum ein. Der Rückstand wird in Ether aufgenommen und mit Wasser und ges. Kochsalzlösung gewaschen. Nach Trocknen über Natriumsulfat und Eindampfen im Vakuum erhält man 0.51 g 7-(2-Benzyloxy-3-cyclopentylphenyl)-6-hydroxy-4-thiaheptansäureethylester.
Rf = 0.38 (LM Cyclohexan/Essigester 2 : 1)
$C_{26}H_{34}O_4S$ MS m/e = 442
$^1$H-NMR (60 MHz, $CDCl_3$, δ ppm) :
1.25 (t, 3H), 1.4-2.2 (m, 8H), 2.4-3.0 (m, 8-9H, 4.15 (q, 2H), 4.86 (s, 2H), 7.0-7.3 (m, 3H), 7.45 (s br, 5H)

Beispiel 26

7-(2-Benzyloxy-3-cyclopentylphenyl)-6-hydroxy-4-thiaheptansäureisopropylester

1 g (2.33 mMol) 7-(2-Benzyloxy-3-cyclopentylphenyl)-6-hydroxy-4-thiaheptansäuremethylester (Beispiel 1) werden in 10 ml Isopropanol gelöst und mit 0.36 ml (1.2 mMol) Titan(IV) isopropylat versetzt. Man erhitzt 6 h unter Feuchtigkeitsausschluß zum sieden, gießt die erkaltete Reaktionsmischung auf 100 ml 2n Salzsäure und extrahiert dreimal mit n-Pentan. Nach Trocknen über Natriumsulfat, Eindampfen im Vakuum und Chromatographie an Kieselgel (LM Cyclohexan/Essigester 3 :1) erhält man 0.8 g 7-(2-Benzyloxy-3-cyclopentylphenyl)-6-hydroxy-4-thiaheptansäureisopropylester.
Rf = 0.39 (LM Cyclohexan/Essigester 2 : 1)
$C_{27}H_{36}O_4S$ MS m/e = 456
$^1$H-NMR (60 MHz, $CDCl_3$, δ ppm) :
1.25 (d, 6H), 1.4-2.2 (m, 8H), 2.4-3.0 (m, 9H), 3.1-3.6 (m, 1H), 3.8-4.2 (m, 1H), 4.90 (s, 2H), 5.03 (quintuplett, 6Hz, 1H), 7.0-7.3 (m, 3H), 7.45 (s br, 5H)

Beispiel 27

4-[5-(2-Benzyloxy-3-cyclopentylphenyl)-4-hydroxy-2-thiapentyl]-1,3-dioxolan-2-on

200 mg (0.48 mMol) 7-(2-Benzyloxy-3-cyclopentylphenyl)-4-thiaheptan-1,2,6-triol (Beispiel 11) werden in 3 ml Tetrahydrofuran gelöst und mit 150 mg (0.93 mMol) N,N'-Carbonyldiimidazol versetzt. Nach 5h Rühren bei 70°C unter Feuchtigkeitsausschluß wird die Lösung mit Ether verdünnt und einmal mit 1n Natronlauge und zweimal mit ges. Kochsalzlösung gewaschen. Trocknen über Natriumsulfat, Abdestillieren des Lösungsmittels in Vakuum und Chromatographie auf Kieselgel (LM Cyclohexan/Essigester 1 : 1) ergibt 133 mg 4-[5-(2-Benzyloxy-3-cyclopentylphenyl)-4-hydroxy-2-thiapentyl]-1,3-dioxolan-2-on.
Rf = 0.34 (LM Cyclohexan/Essigester 1 : 1)
$C_{25}H_{30}O_5S$ MS m/e = 442
$^1$H-NMR (60 MHz, $CDCl_3$, δ ppm) :
1.5-2.1 (m), 2.5-3.0 (m), 3.40 (m, 1H), 4.00 (m, 1H), 4.20 (m, 1H), 4.48 (m, 1H), 4.75 (m, 1H), 4.88 (s, 2H), 7.0-7.5 (m, 8H).

Beispiel 28

7-(3-Cyclopentyl-2-hydroxyphenyl)-6-hydroxy-4-thiaheptansäuremethylester

Diese Verbindung wird analog Beispiel 1e aus (3-Cyclopentyl-2-hydroxybenzyl)oxiran (hergestellt aus Beispiel 1b analog Beispiel 1d) und 3-Mercaptopropionsäuremethylester erhalten.
$C_{18}H_{26}O_4S$ MS m/e = 308
$^1$H-NMR (270 MHz, $CDCl_3$, δ ppm) :
1.5-1.9 (m, 6H), 2.0-2.1 (m, 2H), 2.488 (dd, 15Hz, 11Hz, 1H), 2.614 (t, 7Hz, 2H), 2.75-2.9 (m, 4H), 2.974 (dd,

15Hz, 3Hz, 1H), 3.389 (quintuplett, 8-9Hz, 1H), 3.718 (s, 3H), 3.98-4.08 (m, 1H), 6.802 (t, 8Hz, 1H), 6.873 (dd, 8Hz, 3Hz, 1H), 7.137 (dd, 8Hz, 3Hz, 1H)

Beispiel 29

7-(3-Cyclopentyl-2-hydroxyphenyl)-6-hydroxy-4-thiaheptansäure Natriumsalz

Aus Beipiel 28 durch Verseifung analog Beispiel 21 hergestellt.
$C_{17}H_{24}O_4S$ MS m/e = 294

Beispiel 30

7-(2-Benzyloxy-3-cyclopentylphenyl)-6-hydroxy-4-thiaheptanhydroxamsäure

0.65 g NaOH werden in 3 ml Wasser und 10 ml Methanol gelöst. Man gibt 0.57 g Hydroxylamin-hydrochlorid und dann unter Rühren 1 g 7-(2-Benzyloxy-3-cyclopentylphenyl)-6-hydroxy-4-thiaheptansäuremethylester (Beispiel 1) in 10 ml Methanol gelöst zu. Nachdem eine Stunde bei Raumtemperatur nachgerührt wurde, verdünnt man mit 50 ml Wasser, extrahiert 3x mit Essigester, wäscht die Extrakte mit Wasser und ges. Kochsalzlösung, trocknet über $MgSO_4$ und engt im Vakuum ein. Der Rückstand wird durch Chromatographie an Kieselgel gereinigt (Laufmittel Essigester/Toluol/Pyridin 49 : 49 : 2) Man erhält 0.5 g eines zähen Öls.
Rf = 0.2 (Essigester)
$C_{24}H_{31}NO_4S$ MS (FAB) m/e = 430 (M + $H^+$)
$^1$H-NMR (60 MHz, $CDCl_3$, δ ppm) :
1.3-2.2 (m, 8H), 2.2-3.0 (m,11H), 3.2-3.7 (m, 1H), 3.8-4.2 (m, 1H), 4.88 (s, 2H), 7.0-7.3 (m, 3H), 7.45 (s br, 5H)
analog zu Beispiel 30 werden erhalten :

Beispiel 31

6-(2-Benzyloxy-3-cyclopentylphenyl)-5-hydroxy-3-thiahexanhydroxamsäure

Rf = 0.1 (Essigester/Methanol/Essigsäure 95 : 5 : 1)
$C_{23}H_{29}NO_4S$ MS (DCI) m/e = 416 (M + $H^+$)
$^1$H-NMR (60 MHz, $CDCl_3$, δ ppm) :
1.3-2.2 (m, 8H), 2.2-3.0 (m,7H), 3.2 (s br, 2H), 3.2-3.7 (m, 1H), 3.8-4.2 (m, 1H), 4.87 (s, 2H), 6.9-7.3 (m, 3H), 7.45 (s br, 5H)

Beispiel 32

7-[2-(4-Methoxybenzyloxy)-3-cyclopentylphenyl]-6-hydroxy-4-thiaheptanhydroxamsäure

Rf = 0.23 (Essigester/Essigsäure 98 : 1)
$C_{25}H_{33}NO_5S$ MS (DCI) m/e = 416 (M + $H^+$)
$^1$ H-NMR (60 MHz, $CDCl_3$, δ ppm) :
1.3-2.2 (m, 8H), 2.4-3.0 (m 11H), 3.3-3.6 (m, 1H), 3.83 (s, 3H), 3.8-4.2 (m, 1H), 4.80 (s, 2H), 6.97 (d, 8-9Hz) + 6.8-7.5 (m) + 7.43 (d, 8-9Hz) Σ 7H

Beispiel 33

7-(2-Benzyloxy-3-cyclopentylphenyl)-6-hydroxy-4-oxo-4-thiaheptansäuremethylester.

1 g 7-(2-Benzyloxy-3-cyclopentylphenyl)-6-hydroxy-4-thiaheptansäuremethylester (Beispiel 1) werden in 10 ml THF gelöst. Unter Rühren und Kühlen im Eisbad trägt man portionsweise 0.47 g 3-Chlorperbenzoesäure (techn. 85%-ig) ein. Man läßt 90 Min. bei Raumtemperatur nachrühren, verdünnt mit Essigester, wäscht mit 2n Natronlauge (2x) und ges. Kochsalzlösung, trocknet über $MgSO_4$ und engt im Vakuum ein. Das Rohprodukt wird durch Säulenchromatographie an Kieselgel (Toluol/Essigester 4 : 1 → 2 : 1) gereinigt. Man erhält 0.95 g eines zähen Öls.
Rf = 0.07 (Toluol/Essigester 1 : 1)

$C_{25}H_{32}O_5S$ MS (DCI) m/e = 445 (M + H$^+$)
$^1$H-NMR (60 MHz, $CDCl_3$, δ ppm) :
1.5-2.3 (m, 8H), 2.6-3.1 (m, 9H), 3.2-3.6 (m, 1H), 3.72 (s, 3H), 4.3-4.7 (m, 1H), 4.87 (s, 2H), 7.0-7.4 (m, 3H), 7.45 (s br, 5H)

Beispiel 34

7-(2-Benzyloxy-3-cyclopentylphenyl)-6-hydroxy-4,4-dioxo-4-thiaheptansäuremethylester

1 g 7-(2-Benzyloxy-3-cyclopentylphenyl)-6-hydroxy-4-thiaheptansäuremethylester (Beispiel 1) werden in 10 ml THF gelöst. Unter Rühren und Kühlen im Eisbad trägt man portionsweise 0.95 g 3-Chlorperbenzoesäure (techn. 85%-ig) ein. Man läßt 90 Min. bei Raumtemperatur nachrühren, verdünnt mit Essigester, wäscht mit 2n Natronlauge (2x) und ges. Kochsalzlösung, trocknet über $MgSO_4$ und engt im Vakuum ein. Das Rohprodukt wird durch Säulenchromatographie an Kieselgel (Toluol/Essigester 4 : 1 → 2 : 1) gereinigt. Man erhält 0.90 g eines zähen Öls.
Rf = 0.22 (Toluol/Essigester 1 : 1)
$C_{25}H_{32}O_5S$ MS (DCI) m/e = 460
$^1$H-NMR (60 MHz, $CDCl_3$, δ ppm) :
1.5-2.3 (m, 8H), 2.6-3.1 (m, 5H)), 3.15-3.6 (m, 5H), 3.72 (s, 3H), 4.3-4.7 (m, 1H), 4.88 (s, 2H), 7.0-7.4 (m, 3H), 7.45 (s br, 5H)

Beispiel 35

7-(2-Benzyloxy-3-cyclopentylphenyl)-6-hydroxy-4-thiaheptansäureamid

1 g 7-(2-Benzyloxy-3-cyclopentylphenyl)-6-hydroxy-4-thiaheptansäuremethylester (Beispiel 1) werden in 20 ml einer ca. 10%-igen Lösung von Ammoniak in Methanol gelöst und 7 Tage bei Raumtemperatur im geschlossenen Gefäß stehengelassen. Das Lösungsmittel wird im Vakuum abdestilliert und der Rückstand durch Säulenchromatographie an Kieselgel (Laufmittel Essigester/Cyclohexan 1 : 4) gereinigt.
$C_{24}H_{31}NO_3S$ MS m/e = 413
$^1$H-NMR (60 MHz, $CDCl_3$ δ ppm) :
1.4-2.3 (m, 8H), 2.4-3.0 (m, 9H)), 3.2-3.6 (m, 1H), 4.03 (m, 1H), 4.87 (s, 2H), 4.3-5.8 (m, 2H), 7.0-7.4 (m, 3H), 7.45 (s br, 5H)

Beispiel 36

6-{2-[3-(4-acetyl-3-hydroxy-2-propylphenoxy)propyloxy]-3-cyclopentylphenyl}-5-hydroxy-3-thiahexansäuremethylester

wird analog Beispiel 1 hergestellt.
$C_{31}H_{42}O_7S$ MS m/e = 558
$^1$ H-NMR (270 MHz, $CDCl_3$, δ in ppm) :
0.917 (t, 8Hz, 3H), 1.45-1.6 (m, 8), 1.7-1.8 (m, 2H), 1.9-2.05 (m, 2H), 2.326 (quintuplett, 6Hz, 2H), 2.585 (s, 3H), 2.5-2.75 (m, 4H), 2.84 (d, 7Hz, 2H), 3.268 (s, 2H), 3.15-3.3 (m, 1H), 3.703 (s, 3H), 3.9-4.1 (m, 3H), 4.321 (t, 6Hz, 2H), 6.527 (d, 9Hz, 1H), 7.0-7.1 (m, 2H), 7.13-7.2 (m, 1H), 7.622 (d, 9Hz, 1H), 12.75 (s, 1H)

Beispiel 37

7-(3-Cyclopentyl-2-propargyloxyphenyl)-6-hydroxy-4-thiaheptansäuremethylester

wird analog zu Beispiel 1 hergestellt.
$C_{21}H_{28}O_4S$ MS m/e = 376
$^1$ H-NMR (60 MHz, $CDCl_3$, δ in ppm) :
1.3-2.35 (m, 8H), 2.57 (t, 2.5 Hz), 2.5-2.9 (m), 3.0 (s) Σ 10H, 3.2-3.6 (m, 1H), 3.71 (s, 3H), 4.04 (quintett br., 6Hz, 1H), 4.55 (d, 2.5 Hz, 2H), 7.0-7.5 (m, 3H)

Beispiel 38

7-[3-Cyclopentyl-2-(Oct-2-in-1-yloxy)phenyl]-6-hydroxy-4-thiaheptansäuremethylester

wird analog zu Beispiel 1 hergestellt.
$C_{26}H_{38}O_4S$ MS m/e = 446
$^1$ H-NMR (60 MHz, $CDCl_3$, $\delta$ in ppm) :
0.90 ("t" br, 4-5Hz, 3H), 1.1-2.5 (m, 16H), 2.5-3.0 (m), 3.0 (s) $\Sigma$ 9H, 3.2-3.6 (m, 1H), 3.71 (s, 3H), 4.04 (quintett br., 6Hz, 1H), 4.52 (t, 2 Hz, 2H), 7.0-7.5 (m, 3H)

**Patentansprüche**

**Patentansprüche für folgende Vertragsstaaten : AT, BE, CH, DE, FR, GB, IT, LI, LU, NL, SE**

1. Verbindungen der allgemeinen Formel I :

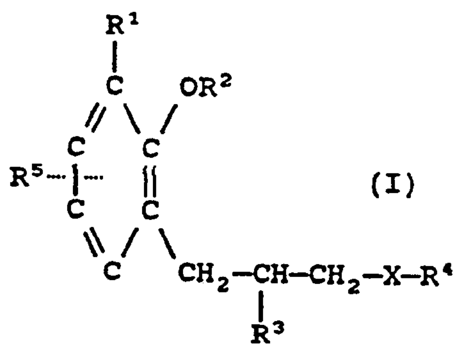

(I)

wobei die Reste folgende Bedeutung haben :

X ist O, S, SO oder $SO_2$ ;

$R^1$ is H, $C_1$-$C_{12}$-Alkyl, $C_2$-$C_{12}$-Alkenyl oder $C_2$-$C_{12}$-Alkinyl (geradkettig oder verzweigt), $C_3$-$C_8$-Cycloalkyl, $C_3$-$C_8$-Cycloalkenyl, Halogen, Phenoxy, $CF_3$, $NO_2$, OH, $OR^6$, COOH, $COOR^6$, CHO oder $COR^7$ ;

$R^2$ is H, $C_1$-$C_{12}$-Alkyl, $C_2$-$C_{12}$-Alkenyl oder $C_2$-$C_{12}$-Alkinyl (geradkettig oder verzweigt), $C_3$-$C_8$-Cycloalkyl, $C_3$-$C_8$-Cycloalkenyl, Phenyl-$C_1$-$C_{10}$-alkyl, Phenoxy-$C_1$-$C_{10}$-alkyl, Phenyl-$C_2$-$C_{10}$-alkenyl oder Phenyl-$C_2$-$C_{10}$-alkinyl, wobei die Phenylringe noch durch 1-3 $C_1$-$C_4$-Alkyl, $C_2$-$C_4$-Alkenyl, $C_1$-$C_4$-Alkoxy, $C_1$-$C_4$-Alkanoyl, $C_1$-$C_4$-Alkoxycarbonyl, Hydroxy oder Halogenreste substituiert sein können, oder $R^2$ ist Pyridylmethyl oder Thienylmethyl ;

$R^3$ ist OH, $OR^6$, $OCOR^7$ ;

$R^4$ ist $C_1$-$C_4$-Alkyl, $C_2$-$C_4$-Hydroxyalkyl, $C_3$-$C_4$-Dihydroxyalkyl, $C_1$-$C_4$-Alkoxy-$C_1$-$C_4$-alkyl, $C_1$-$C_4$-Alkanoyloxy-$C_1$-$C_4$-alkyl, Phenyl oder Phenyl-$C_1$-$C_4$-alkyl, wobei die Phenylringe mit HO, Halogen, $C_1$-$C_4$-Alkoxy oder $C_1$-$C_4$-Alkylthio ein- oder zweifach substituiert sein können, oder $R^4$ ist eine Gruppe der allgemeinen Formel $(CH_2)_nCOR^8$ oder $(CH_2)_nR^9$ ;

$R^5$ ist H, Halogen, $CF_3$, OH, $C_1$-$C_4$-Alkyl oder $C_1$-$C_4$-Alkoxy ;

$R^6$ ist $C_1$-$C_4$-Alkyl, Allyl oder Benzyl ;

$R^7$ ist $C_1$-$C_4$-Alkyl oder Phenyl ;

$R^8$ ist OH, $C_1$-$C_4$-Alkoxy, $CH_2OH$, $OCH_2CO_2H$, $OCH_2CO_2R^7$, $OCH_2Ph$, NHOH, $NH_2$, $NHR^7$, $NR^7_2$, Piperidino, Pyrrolidino, Morpholino oder Phenoxy, wobei der Phenoxyrest noch durch Carboxy, $C_1$-$C_4$-Alkoxycarbonyl, OH oder $OCH_3$ substituiert sein kann ;

$R^9$ ist Tetrazol-5-yl oder 2-Oxo-1,3-dioxolan-4-yl ;

n ist 0, 1, 2 oder 3 ;

sowie physiologisch verträgliche Salze solcher Verbindungen der allgemeinen Formel I, in denen einer der Reste eine Carboxylgruppe (COOH) enthält.

2. Verbindungen laut Anspruch 1 der allgemeinen Formel I, in der die Reste die folgende Bedeutung haben:

X ist O, S, SO oder $SO_2$ ;

$R^1$ ist H, $C_1$-$C_4$-Alkyl, $C_3$-$C_8$-Cycloalkyl oder $C_3$-$C_8$-Cycloalkenyl ;

$R^2$ is H, $C_1$-$C_{10}$-Alkyl, (geradkettig oder verzweigt), $C_3$-$C_8$-Cycloalkyl, Phenyl-$C_1$-$C_{10}$-alkyl oder Phenoxy-

$C_1$-$C_{10}$-alkyl, wobei die Phenylringe noch durch 1-3 $C_1$-$C_4$-Alkyl, $C_2$-$C_4$-Alkenyl, $C_1$-$C_4$-Alkoxy, $C_1$-$C_4$-Alkanoyl, $C_1$-$C_4$-Alkoxycarbonyl, Hydroxy oder Halogenreste substituiert sein können, oder $R^2$ ist Pyridylmethyl oder Thienylmethyl ;

$R^3$ ist OH ;

$R^4$ ist $C_1$-$C_4$-Alkyl, $C_2$-$C_4$-Hydroxyalkyl, $C_3$-$C_4$-Dihydroxyalkyl, $C_1$-$C_4$-Alkoxy-$C_1$-$C_4$-alkyl, $C_1$-$C_4$-Alkanoyloxy-$C_1$-$C_4$-alkyl, Phenyl oder Phenyl-$C_1$-$C_4$-alkyl, wobei die Phenylringe mit HO, Halogen, $C_1$-$C_4$-Alkoxy oder $C_1$-$C_4$-Alkylthio ein- oder zweifach substituiert sein können, oder $R^4$ ist eine Gruppe der allgemeinen Formel $(CH_2)_nCOR^8$ oder $(CH_2)_nR^9$ ;

$R^5$ ist H oder Halogen ;

$R^7$ ist $C_1$-$C_4$-Alkyl ;

$R^8$ ist OH, $C_1$-$C_4$-Alkoxy, $CH_2OH$, $OCH_2CO_2H$, $OCH_2CO_2R^7$, $OCH_2Ph$, NHOH, $NH_2$, $NHR^7$, $NR^7_2$, Piperidino, Pyrrolidino, Morpholino, oder Phenoxy, wobei der Phenoxyrest noch durch Carboxy, $C_1$-$C_4$-Alkoxycarbonyl, OH oder $OCH_3$ substituiert sein kann ;

$R^9$ ist Tetrazol-5-yl ;

n ist 1, 2 oder 3.

3. Verbindungen laut Anspruch 1 der allgemeinen Formel I, in der die Reste die folgende Bedeutung haben:

X ist S ;

$R^1$ ist H, $CH_3$ oder Cyclopentyl ;

$R^2$ ist H, Benzyl, wobei der Benzylrest am Phenylring noch durch Methoxy oder Chlor ein- oder zweifach substituiert sein kann, Phenoxy-$C_2$-$C_4$-alkyl, wobei der Phenylring noch mit 1-3 $C_1$-$C_4$-Alkyl, $C_1$-$C_4$-Alkanoyl oder Hydroxygruppen substituiert sein kann, Pyridylmethyl oder Thienylmethyl ;

$R^3$ ist OH ;

$R^4$ ist $C_1$-$C_4$-Alkyl, $C_2$-$C_4$-Hydroxyalkyl, $C_3$-$C_4$-Dihydroxyalkyl, $C_1$-$C_4$-Alkoxy-$C_1$-$C_4$-alkyl, $C_1$-$C_4$-Alkanoyloxy-$C_1$-$C_4$-alkyl, Phenyl oder Benzyl, wobei die Phenylringe mit HO, Methoxy, oder Methylthio ein- oder zweifach substituiert sein können, oder $R^4$ ist eine Gruppe der allgemeinen Formel $(CH_2)_nCOR^8$ oder $(CH_2)_nR^9$ ;

$R^5$ ist H oder Chlor ;

$R^7$ ist $C_1$-$C_4$-Alkyl ;

$R^8$ ist OH, $C_1$-$C_4$-Alkoxy, $CH_2OH$, $OCH_2CO_2H$, $OCH_2CO_2R^7$, $OCH_2Ph$, NHOH, $NH_2$, Piperidino, Pyrrolidino, Morpholino, oder Phenoxy, wobei der Phenoxyrest noch durch Carboxy, $C_1$-$C_4$-Alkoxycarbonyl, OH oder $OCH_3$ substituiert sein kann ;

$R^9$ ist Tetrazol-5-yl ;

n ist 1, 2 oder 3.

4. (6RS)-7-(2-Benzyloxyphenyl)-6-hydroxy-4-thiaheptansäuremethylester

5. (6RS)-7-(2-Benzyloxy3-cyclopentylphenyl)-6-hydroxy-4-thiaheptansäuremethylester

6. (6RS)-7-(2-Benzyloxy-3-methylphenyl)-6-hydroxy-4-thiaheptansäuremethylester

7. (6RS)-7-(2-Benzyloxy-3,5-dimethylphenyl)-6-hydroxy-4-thiaheptansäuremethylester

8. (6RS)-7-(2-Benzyloxy-3-cyclopentylphenyl)-6-hydroxy-4-thiaheptansäure

9. (6RS)-7-(2-Benzyloxy-5-chlorphenyl)-6-hydroxy-4-thiaheptan-säuremethylester

10. (5RS)-6-(2-Benzyloxy-3-cyclopentylphenyl)-3-thiahexan-1,5-diol

11. Verfahren zur Herstellung der Verbindungen nach Anspruch 1, dadurch gekennzeichnet, daß man A) eine Verbindung der allgemeinen Formel II,

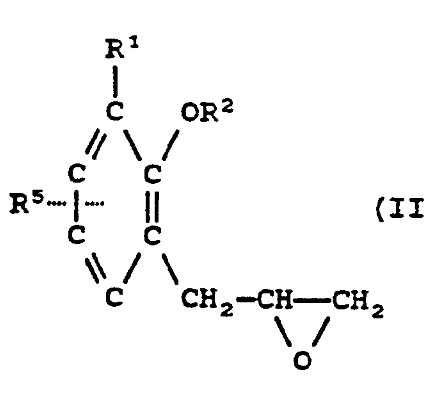

(II)

in der die Reste $R^1$, $R^2$ und $R^5$ die in Anspruch 1 genannte Bedeutung haben, mit einer Verbindung der allgemeinen Formel $R^4XH$, wobei $R^4$ die in Anspruch 1 genannte Bedeutung hat und X O oder S ist, zu einer Verbindung der Formel I umsetzt, worin $R^1$, $R^2$, $R^4$ und $R^5$ die angegebene Bedeutung haben, $R^3$ die

Hydroxygruppe ist und X Schwefel oder Sauerstoff bedeutet, oder B) eine Verbindung der allgemeinen Formel III,

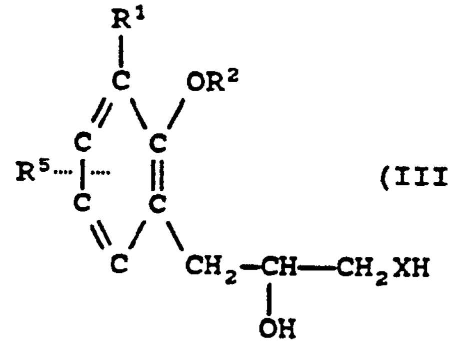

(III)

in der die Reste $R^1$, $R^2$ und $R^5$ die in Anspruch 1 genannte Bedeutung haben, und X O oder S bedeuted, mit einer Verbindung der allgemeinen Formel $R^4Y$, wobei $R^4$ die in Anspruch 1 genannte Bedeutung hat und Y eine Abgangsgruppe ist, zu einer Verbindung der Formel I umsetzt, worin $R^1$, $R^2$, $R^4$ und $R^5$ die angegebene Bedeutung haben, $R^3$ die Hydroxygruppe ist und X Schwefel oder Sauerstoff bedeutet, und gegebenenfalls eine erhaltene Verbindung durch Umwandlung in andere Verbindungen der Formel I überführt.

12. Pharmazeutische Zubereitungen, die eine oder mehrere der Verbindungen gemäß Anspruch 1 enthalten.

13. Eine Verbindung gemäß Anspruch 1 zur Behandlung von Krankheiten, die durch erhöhte Spiegel von Leukotrienen gekennzeichnet sind, wie Asthma, allergische Hautreaktionen, Psoriasis, ulcerativer Colitis, rheumatischer Arthritis und Schock.

14. Eine pharmazeutische Zubereitung gemäß Anspruch 12 zur Behandlung von Krankheiten, die durch erhöhte Spiegel von Leukotrienen gekennzeichnet sind, wie Asthma, allergische Hautreaktionen, Psoriasis, ulcerativer Colitis, rheumatischer Arthritis und Schock.

**Patentansprüche für folgende Vertragsstaaten : ES, GR**

1. Verfahren zur Herstellung von Verbindungen der allgemeinen Formel I :

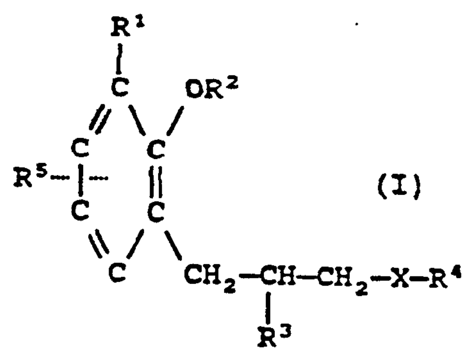

(I)

wobei die Reste folgende Bedeutung haben :

X ist O, S, SO oder $SO_2$ ;

$R^1$ ist H, $C_1$-$C_{12}$-Alkyl, $C_2$-$C_{12}$-Alkenyl oder $C_2$-$C_{12}$-Alkinyl (geradkettig oder verzweigt), $C_3$-$C_8$-Cycloalkyl, $C_3$-$C_8$-Cycloalkenyl, Halogen, Phenoxy, $CF_3$, $NO_2$, OH, $OR^6$, COOH, $COOR^6$, CHO oder $COR^7$ ;

$R^2$ is H, $C_1$-$C_{12}$-Alkyl, $C_2$-$C_{12}$-Alkenyl oder $C_2$-$C_{12}$-Alkinyl (geradkettig oder verzweigt), $C_3$-$C_8$-Cycloalkyl, $C_3$-$C_8$-Cycloalkenyl, Phenyl-$C_1$-$C_{10}$-alkyl, Phenoxy-$C_1$-$C_{10}$-alkyl, Phenyl-$C_2$-$C_{10}$-alkenyl oder Phenyl-$C_2$-$C_{10}$-alkinyl, wobei die Phenylringe noch durch 1-3 $C_1$-$C_4$-Alkyl, $C_2$-$C_4$-Alkenyl, $C_1$-$C_4$-Alkoxy, $C_1$-$C_4$-Alkanoyl, $C_1$-$C_4$-Alkoxycarbonyl, Hydroxy oder Halogenreste substituiert sein können, oder $R^2$ ist Pyridylmethyl oder Thienylmethyl ;

$R^3$ ist OH, $OR^6$, $OCOR^7$ ;

$R^4$ ist $C_1$-$C_4$-Alkyl, $C_2$-$C_4$-Hydroxyalkyl, $C_3$-$C_4$-Dihydroxyalkyl, $C_1$-$C_4$-Alkoxy-$C_1$-$C_4$-Alkyl, $C_1$-$C_4$- Alkanoylo-

xy-$C_1$-$C_4$-alkyl, Phenyl oder Phenyl-$C_1$-$C_4$-alkyl, wobei die Phenylringe mit HO, Halogen, $C_1$-$C_4$-Alkoxy oder $C_1$-$C_4$-Alkylthio ein- oder zweifach substituiert sein können, oder $R^4$ ist eine Gruppe der allgemeinen Formel $(CH_2)_nCOR^8$ oder $(CH_2)_nR^9$ ;

$R^5$ ist H, Halogen, $CF_3$, OH, $C_1$-$C_4$-Alkyl oder $C_1$-$C_4$-Alkoxy ;

$R^6$ ist $C_1$-$C_4$-Alkyl, Allyl oder Benzyl ;

$R^7$ ist $C_1$-$C_4$-Alkyl oder Phenyl ;

$R^8$ ist OH, $C_1$-$C_4$-Alkoxy, $CH_2OH$, $OCH_2CO_2H$, $OCH_2CO_2R^7$, $OCH_2Ph$, NHOH, $NH_2$, $NHR^7$, $NR^7_2$, Piperidino, Pyrrolidino, Morpholino oder Phenoxy, wobei der Phenoxyrest noch durch Carboxy, $C_1$-$C_4$-Alkoxycarbonyl, OH oder $OCH_3$ substituiert sein kann ;

$R^9$ ist Tetrazol-5-yl oder 2-Oxo-1,3-dioxolan-4-yl ;

n ist 0, 1, 2 oder 3 ;

sowie von physiologisch verträglichen Salzen solcher Verbindungen der allgemeinen Formel I, in denen einer der Reste eine Carboxylgruppe (COOH) enthält, dadurch gekennzeichnet, daß man A) eine Verbindung der allgemeinen Formel II,

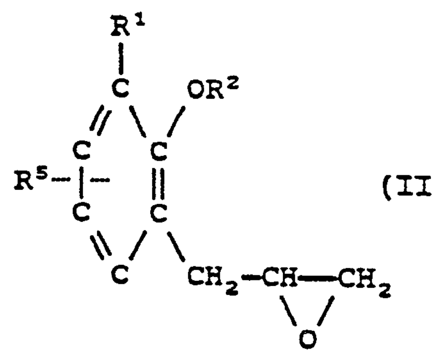

(II)

in der die Reste $R^1$, $R^2$ und $R^5$ die zu Formel I genannte Bedeutung haben, mit einer Verbindung der allgemeinen Formel $R^4XH$, wobei $R^4$ die zu Formel I genannte Bedeutung hat und X O oder S ist, zu einer Verbindung der Formel I umsetzt, worin $R^1$, $R^2$, $R^4$ und $R^5$ die angegebene Bedeutung haben, $R^3$ die Hydroxygruppe ist und X Schwefel oder Sauerstoff bedeutet, oder B) eine Verbindung der allgemeinen Formel III,

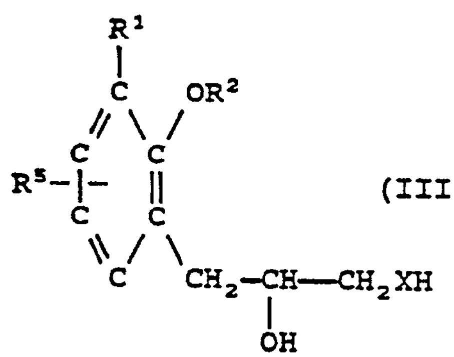

(III)

in der die Reste $R^1$, $R^2$ und $R^5$ die zu Formel I genannte Bedeutung haben, und X O oder S bedeutet, mit einer Verbindung der allgemeinen Formel $R^4Y$, wobei $R^4$ die zu Formel I genannte Bedeutung hat und Y eine Abgangsgruppe ist, zu einer Verbindung der Formel I umsetzt, worin $R^1$, $R^2$, $R^4$ und $R^5$ die angegebene Bedeutung haben, $R^3$ die Hydroxygruppe ist und X Schwefel oder Sauerstoff bedeutet, und gegebenenfalls eine erhaltene Verbindung durch Umwandlung in andere Verbindungen der Formel I überführt.

2. Verfahren gemäß Anspruch 1, dadurch gekennzeichnet, daß man eine Verbindung der allgemeinen Formel I, in der die Reste die folgende Bedeutung haben :

X ist O, S, SO oder $SO_2$ ;

$R^1$ ist H, $C_1$-$C_4$-Alkyl, $C_3$-$C_8$-Cycloalkyl oder $C_3$-$C_8$-Cycloalkenyl ;

$R^2$ is H, $C_1$-$C_{10}$-Alkyl, (geradkettig oder verzweigt), $C_3$-$C_8$-Cycloalkyl, Phenyl-$C_1$-$C_{10}$-alkyl oder Phenoxy-$C_1$-$C_{10}$-alkyl, wobei die Phenylringe noch durch 1-3 $C_1$-$C_4$-Alkyl, $C_2$-$C_4$-Alkenyl, $C_1$-$C_4$-Alkoxy, $C_1$-$C_4$-Alkanoyl,

$C_1$-$C_4$-Alkoxycarbonyl, Hydroxy oder Halogenreste substituiert sein können, oder $R^2$ ist Pyridylmethyl oder Thienylmethyl ;

$R^3$ ist OH ;

$R^4$ ist $C_1$-$C_4$-Alkyl, $C_2$-$C_4$-Hydroxyalkyl, $C_3$-$C_4$-Dihydroxyalkyl, $C_1$-$C_4$-Alkoxy-$C_1$-$C_4$-alkyl, $C_1$-$C_4$-Alkanoyloxy-$C_1$-$C_4$-alkyl, Phenyl oder Phenyl-$C_1$-$C_4$-alkyl, wobei die Phenylringe mit HO, Halogen, $C_1$-$C_4$-Alkoxy oder $C_1$-$C_4$-Alkylthio ein- oder zweifach substituiert sein können, oder $R^4$ ist eine Gruppe der allgemeinen Formel $(CH_2)_nCOR^8$ oder $(CH_2)_nR^9$ ;

$R^5$ ist H oder Halogen ;

$R^7$ ist $C_1$-$C_4$-Alkyl ;

$R^8$ ist OH, $C_1$-$C_4$-Alkoxy, $CH_2OH$, $OCH_2CO_2H$, $OCH_2CO_2R^7$, $OCH_2Ph$, NHOH, $NH_2$, $NHR^7$, $NR^7_2$, Piperidino, Pyrrolidino, Morpholino oder Phenoxy, wobei der Phenoxyrest noch durch Carboxy, $C_1$-$C_4$-Alkoxycarbonyl, OH oder $OCH_3$ substituiert sein kann ;

$R^9$ ist Tetrazol-5-yl ;

n ist 1, 2 oder 3, herstellt.

3. Verfahren gemäß Anspruch 1, dadurch gekennzeichnet, daß man eine Verbindung der allgemeinen Formel I, in der die Reste die folgende Bedeutung haben :

X ist S ;

$R^1$ ist H, $CH_3$ oder Cyclopentyl ;

$R^2$ ist H, Benzyl, wobei der Benzylrest am Phenylring noch durch Methoxy oder Chlor ein- oder zweifach substituiert sein kann, Phenoxy-$C_2$-$C_4$-alkyl, wobei der Phenylring noch mit 1-3 $C_1$-$C_4$-Alkyl, $C_1$-$C_4$-Alkanoyl oder Hydroxygruppen substituiert sein kann, Pyridylmethyl oder Thienylmethyl ;

$R^3$ ist OH ;

$R^4$ ist $C_1$-$C_4$-Alkyl, $C_2$-$C_4$-Hydroxyalkyl, $C_3$-$C_4$-Dihydroxyalkyl, $C_1$-$C_4$-Alkoxy-$C_1$-$C_4$-alkyl, $C_1$-$C_4$-Alkanoyloxy-$C_1$-$C_4$-alkyl, Phenyl oder Benzyl, wobei die Phenylringe mit HO, Methoxy oder Methylthio ein- oder zweifach substituiert sein können, oder $R^4$ ist eine Gruppe der allgemeinen Formel $(CH_2)_nCOR^8$ oder $(CH_2)_nR^9$ ;

$R^5$ ist H oder Chlor ;

$R^7$ ist $C_1$-$C_4$-Alkyl ;

$R^8$ ist OH, $C_1$-$C_4$-Alkoxy, $CH_2OH$, $OCH_2CO_2H$, $OCH_2CO_2R^7$, $OCH_2Ph$, NHOH, $NH_2$, Piperidino, Pyrrolidino, Morpholino oder Phenoxy, wobei der Phenoxyrest noch durch Carboxy, $C_1$-$C_4$-Alkoxycarbonyl, OH oder $OCH_3$ substituiert sein kann ;

$R^9$ ist Tetrazol-5-yl ;

n ist 1, 2 oder 3, herstellt.

4. Verfahren gemäß Anspruch 1, dadurch gekennzeichnet, daß man den (6RS)-7-(2-Benzyloxyphenyl)-6-hydroxy-4-thiaheptansäuremethylester herstellt.

5. Verfahren gemäß Anspruch 1, dadurch gekennzeichnet, daß man den (6RS)-7-(2-Benzyloxy-3-cyclopentylphenyl)-6-hydroxy-4-thiaheptansäuremethylester herstellt.

6. Verfahren gemäß Anspruch 1, dadurch gekennzeichnet, daß man den (6RS)-7-(2-Benzyloxy-3-methylphenyl)-6-hydroxy-4-thiaheptansäuremethylester herstellt.

7. Verfahren gemäß Anspruch 1, dadurch gekennzeichnet, daß man den (6RS)-7-(2-Benzyloxy-3,5-dimethylphenyl)-6-hydroxy-4-thiaheptansäuremethylester herstellt.

8. Verfahren gemäß Anspruch 1, dadurch gekennzeichnet, daß man die (6RS)-7-(2-Benzyloxy-3-cyclopentylphenyl)-6-hydroxy-4-thiaheptansäure herstellt.

9. Verfahren gemäß Anspruch 1, dadurch gekennzeichnet, daß man den (6RS)-7-(2-Benzyloxy-5-chlorphenyl)-6-hydroxy-4-thiaheptan-säuremethylester herstellt.

10. Verfahren gemäß Anspruch 1, dadurch gekennzeichnet, daß man den (5RS)-6-(2-Benzyloxy-3-cyclopentylphenyl)-3-thiahexan-1,5-diol herstellt.

11. Verfahren zur Herstellung einer pharmazeutischen Zubereitung, dadurch gekennzeichnet, daß man eine oder mehrere der Verbindungen, erhältlich gemäß Anspruch 1, in eine geeignete Darreichungsform bringt.

## Claims

**Claims for the following Contracting States : AT, BE, CH, DE, FR, GB, IT, LI, LU, NL, SE**

1. A compound of the formula I :

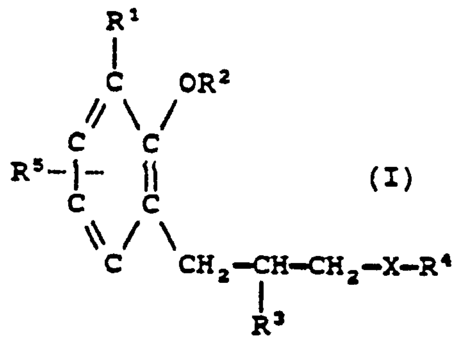

(I)

where the radicals have the following meaning :

X is O, S, SO, or $SO_2$ ;

$R^1$ is H, $C_1$-$C_{12}$-alkyl, $C_2$-$C_{12}$-alkenyl or $C_2$-$C_{12}$-alkyl(straight-chain or branched), $C_3$-$C_8$-cycloalkyl, $C_3$-$C_8$-cycloalkenyl, halogen, phenoxy, $CF_3$, $NO_2$, OH, $OR^6$, COOH, $COOR^6$, CHO or $COR^7$ ;

$R^2$ is H, $C_1$-$C_{12}$-alkyl, $C_2$-$C_{12}$-alkenyl or $C_2$-$C_{12}$-alkynyl (straight-chain or branched), $C_3$-$C_8$-cycloalkyl, $C_3$-$C_8$-cycloalkenyl, phenyl-$C_1$-$C_{10}$-alkyl, phenoxy-$C_1$-$C_{10}$-alkyl, phenyl-$C_2$-$C_{10}$-alkenyl or phenyl-$C_2$-$C_{10}$-alkynyl, it being possible for the phenyl rings also to be substituted by 1-3 $C_1$-$C_4$-alkyl, $C_2$-$C_4$-alkenyl, $C_1$-$C_4$-alkoxy, $C_1$-$C_4$-alkanoyl, $C_1$-$C_4$-alkoxycarbonyl, hydroxyl or halogen radicals, or $R^2$ is pyridylmethyl or thienylmethyl ;

$R^3$ is OH, $OR^6$ or $OCOR^7$ ;

$R^4$ is $C_1$-$C_4$-alkyl, $C_2$-$C_4$-hydroxyalkyl, $C_3$-$C_4$-dihydroxyalkyl, $C_1$-$C_4$-alkoxy-$C_1$-$C_4$-alkyl, $C_1$-$C_4$-alkanoyloxy-$C_1$-$C_4$-alkyl, phenyl or phenyl-$C_1$-$C_4$-alkyl, it being possible for the phenyl rings to be substituted once or twice by HO ; halogen, $C_1$-$C_4$-alkoxy or $C_1$-$C_4$-alkylthio, or $R^4$ is a group of the general formula $(CH_2)_nCOR^8$ or $(CH_2)_nR^9$ ;

$R^5$ is H, halogen, $CF_3$, OH, $C_1$-$C_4$-alkyl or $C_1$-$C_4$-alkoxy ;

$R^6$ is $C_1$-$C_4$-alkyl, allyl or benzyl ;

$R^7$ is $C_1$-$C_4$-alkyl or phenyl ;

$R^8$ is OH, $C_1$-$C_4$-alkoxy, $CH_2OH$, $OCH_2CO_2H$, $OCH_2CO_2R^7$, $OCH_2Ph$, NHOH, $NH_2$, $NHR^7$, $NR^7{}_2$, piperidino, pyrrolidino, morpholino or phenoxy, it being possible for the phenoxy radical also to be substituted by carboxyl, $C_1$-$C_4$-alkoxycarbonyl, OH or $OCH_3$ ;

$R^9$ is tetrazol-5-yl or 2-oxo-1,3-dioxolan-4-yl ;

n is 0, 1, 2 or 3 ;

as well as physiologically tolerated salts of a compound of the formula I in which one of the radicals contains a carboxyl group (COOH).

2. A compound as clawed in claim 1 and of the formula I, in which the radicals have the following meaning:

X is O, S, SO or $SO_2$ ;

$R^1$ is H, $C_1$-$C_4$-alkyl, $C_3$-$C_8$-cycloalkyl or $C_3$-$C_8$-cycloalkenyl ;

$R^2$ is H, $C_1$-$C_{10}$-alkyl (straight-chain or branched) ; $C_3$-$C_8$-cycloalkyl, phenyl-$C_1$-$C_{10}$-alkyl or phenoxy-$C_1$-$C_{10}$-alkyl, it being possible for the phenyl rings also to be substituted by 1-3 $C_1$-$C_4$-alkyl, $C_2$-$C_4$-alkenyl, $C_1$-$C_4$-alkoxy, $C_1$-$C_4$-alkanoyl, $C_1$-$C_4$-alkoxycarbonyl, hydroxyl or halogen radicals,or $R^2$ is pyridylmethyl or thienylmethyl ;

$R^3$ is OH ;

$R^4$ is $C_1$-$C_4$-alkyl, $C_2$-$C_4$-hydroxyalkyl, $C_3$-$C_4$-dihydroxyalkyl, $C_1$-$C_4$-alkoxy-$C_1$-$C_4$-alkyl, $C_1$-$C_4$-alkanoyloxy-$C_1$-$C_4$-alkyl, phenyl or phenyl-$C_1$-$C_4$-alkyl, it being possible for the phenyl rings to be substituted once or twice by HO, halogen, $C_1$-$C_4$-alkoxy or $C_1$-$C_4$-alkylthio, or $R^4$ is a group of the general formula $(CH_2)_nCOR^8$ or $(CH_2)_nR^9$ ;

$R^6$ is H or halogen ;

$R^7$ is $C_1$-$C_4$-alkyl ;

$R^8$ is OH, $C_1$-$C_4$-alkoxy, $CH_2OH$, $OCH_2CO_2H$, $OCH_2CO_2R^7$, $OCH_2Ph$, NHOH, $NH_2$, $NHR^7$, $NR^7{}_2$, piperidino, pyrrolidino, morpholino, or phenoxy, it being possible for the phenoxy radical also to be substituted by carboxyl, $C_1$-$C_4$-alkoxycarbonyl, OH or $OCH_3$ ;

$R^9$ is tetrazol-5-yl ;

n is 1, 2 or 3.

3. A compound as claimed in claim 1 and of the formula I, in which the radicals have the following meaning:

X is S ;

$R^1$ is H, $CH_3$ or cyclopentyl ;

$R^2$ is H, benzyl, it being possible for the benzyl radical also to be substituted once or twice on the phenyl

ring by methoxy or chlorine, is phenoxy-$C_2$-$C_4$-alkyl, it being possible for the phenyl ring also to be substituted by 1-3 $C_1$-$C_4$-alkyl, $C_1$-$C_4$-alkanoyl or hydroxy groups ; or is pyridylmethyl or thienylmethyl ;

$R^3$ is OH ;

$R^4$ is $C_1$-$C_4$-alkyl, $C_2$-$C_4$-hydroxyalkyl, $C_3$-$C_4$-dihydroxyalkyl, $C_1$-$C_4$-alkoxy-$C_1$-$C_4$-alkyl, $C_1$-$C_4$-alkanoyloxy-$C_1$-$C_4$-alkyl, phenyl or benzyl, it being possible for the phenyl rings to be substituted once or twice by HO, methoxy or methylthio ; or $R^4$ is a group of the general formula $(CH_2)_nCOR^8$ or $(CH_2)_nR^9$ ;

$R^5$ is H or chlorine ;

$R^7$ is $C_1$-$C_4$-alkyl ;

$R^8$ is OH, $C_1$-$C_4$-alkoxy, $CH_2OH$, $OCH_2CO_2H$, $OCH_2CO_2R^7$, $OCH_2Ph$, NHOH, $NH_2$, piperidino, pyrrolidino, morpholino, or phenoxy, it being possible for the phenoxy radical also to be substituted by carboxyl, $C_1$-$C_4$-alkoxycarbonyl, OH or $OCH_3$ ;

$R^9$ is tetrazol-5-yl ;

n is 1, 2 or 3.

4. Methyl (6RS)-7-(2-benzyloxyphenyl)-6-hydroxy-4-thiaheptanoate
5. Methyl (6RS)-7-(2-benzyloxy-3-cyclopentylphenyl)-6-hydroxy-4-thiaheptanoate
6. Methyl (6RS)-7-(2-benzyloxy-3-methylphenyl)-6-hydroxy-4-thiaheptanoate
7. Methyl (6RS)-7-(2-benzyloxy-3,5-dimethylphenyl)-6-hydroxy-4-thiaheptanoate
8. (6RS)-7-(2-Benzyloxy-3-cyclopentylphenyl)-6-hydroxy-4-thiaheptanoic acid
9. Methyl (6RS)-7-(2-benzyloxy-5-chlorophenyl)-6-hydroxy-4-thiaheptanoate
10. (5RS)-6-(2-Benzyloxy-3-cyclopentylphenyl)-3-thiahexane-1,5,-diol
11. A process for the preparation of a compound as claimed in claim 1, which comprises A) reacting a compound of the formula II

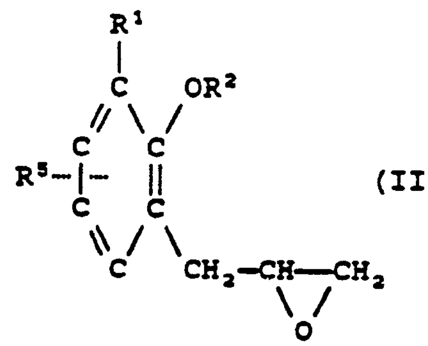

(II)

in which the radicals $R^1$, $R^2$ and $R^5$ have the meaning mentioned in claim 1, with a compound of the formula $R^4XH$, where $R^4$ has the meaning mentioned in claim 1, and X is O or S, to give a compound of the formula I in which $R^1$, $R^2$, $R^4$ and $R^5$ have the indicated meaning, $R^3$ is the hydroxyl group, and X denotes sulfur or oxygen, or B) reacting a compound of the formula III

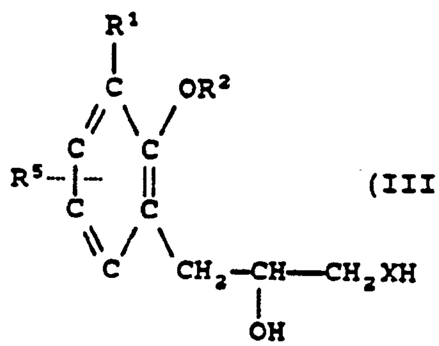

(III)

in which the radicals $R^1$, $R^2$ and $R^5$ have the meaning mentioned in claim 1, and X denotes O or S, with a compound of the formula $R^4Y$, where $R^4$ has the meaning mentioned in claim 1, and Y is a leaving group to give a compound of the formula I in which $R^1$, $R^2$, $R^4$ and $R^5$ have the indicated meaning, $R^3$ is the hydroxyl group and X denotes sulfur or oxygen, and, where appropriate, transforming a resulting compound by conversion into

other compounds of the formula I.

12. A pharmaceutical composition which contains one or more compounds as claimed in claim 1.

13. A compound as claimed in claim 1 for the treatment of disorders associated with elevated levels of leukotrienes, such as asthma, allergic skin reactions, psoriasis, ulcerative colitis, rheumatoid arthritis and shock.

14. A pharmaceutical composition as claimed in claim 12 for the treatment of disorders associated with elevated levels of leukotrienes, such as asthma, allergic skin reactions, psoriasis, ulcerative colitis, rheumatoid arthritis and shock.

**Claims for the following Contracting States : ES, GR**

1. A process for the preparation compound of the formula I :

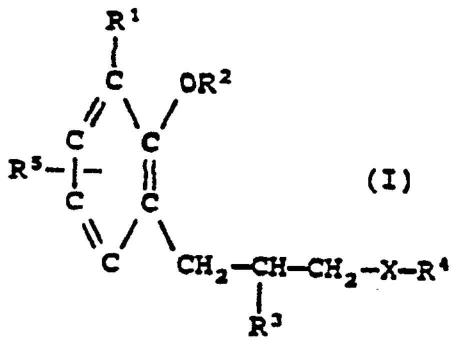

(I)

where the radicals have the following meaning :

X is O, S, SO, or $SO_2$ ;

$R^1$ is H, $C_1$-$C_{12}$-alkyl, $C_2$-$C_{12}$-alkenyl or $C_2$-$C_{12}$-alkynyl (straight-chain or branched), $C_3$-$C_8$-cycloalkyl, $C_3$-$C_8$-cycloalkenyl, halogen, phenoxy, $CF_3$, $NO_2$, OH, $OR^6$, COOH, $COOR^6$, CHO or $COR^7$ ;

$R^2$ is H, $C_1$-$C_{12}$-alkyl, $C_2$-$C_{12}$-alkenyl or $C_2$-$C_{12}$-alkynyl (straight-chain or branched), $C_3$-$C_8$-cycloalkyl, $C_3$-$C_8$-cycloalkenyl, phenyl-$C_1$-$C_{10}$-alkyl, phenoxy-$C_1$-$C_{10}$-alkyl, phenyl-$C_2$-$C_{10}$-alkenyl or phenyl-$C_2$-$C_{10}$-alkynyl, it being possible for the phenyl rings also to be substituted by 1-3 $C_1$-$C_4$-alkyl, $C_2$-$C_4$-alkenyl, $C_1$-$C_4$-alkoxy, $C_1$-$C_4$-alkanoyl, $C_1$-$C_4$-alkoxycarbonyl, hydroxyl or halogen radicals, or $R^2$ is pyridylmethyl or thienylmethyl ;

$R^3$ is OH, $OR^6$ or $OCOR^7$ ;

$R^4$ is $C_1$-$C_4$-alkyl, $C_2$-$C_4$-hydroxyalkyl, $C_3$-$C_4$-dihydroxyalkyl, $C_1$-$C_4$-alkoxy-$C_1$-$C_4$-alkyl, $C_1$-$C_4$-alkanoyloxy-$C_1$-$C_4$-alkyl, phenyl or phenyl-$C_1$-$C_4$-alkyl, it being possible for the phenyl rings to be substituted once or twice by HO, halogen, $C_1$-$C_4$-alkoxy or $C_1$-$C_4$-alkylthio, or $R^4$ is a group of the general formula $(CH_2)_nCOR^8$ or $(CH_2)_nR^9$ ;

$R^5$ is H, halogen, $CF_3$, OH, $C_1$-$C_4$-alkyl or $C_1$-$C_4$-alkoxy ;

$R^6$ is $C_1$-$C_4$-alkyl, allyl or benzyl ;

$R^7$ is $C_1$-$C_4$-alkyl or phenyl ;

$R^8$ is OH, $C_1$-$C_4$-alkoxy, $CH_2OH$, $OCH_2CO_2H$, $OCH_2CO_2R^7$, $OCH_2Ph$, NHOH, $NH_2$, $NHR^7$, $NR^7_2$, piperidino, pyrrolidino, morpholino or phenoxy, it being possible for the phenoxy radical also to be substituted by carboxyl, $C_1$-$C_4$-alkoxycarbonyl, OH or $OCH_3$ ;

$R^9$ is tetrazol-5-yl or 2-oxo-1,3-dioxolan-4-yl ;

n is 0, 1, 2 or 3 ;

as well as physiologically tolerated salts of a compound of the formula I in which one of the radicals contains a carboxyl group (COOH), which comprises A) reacting a compound of the formula II

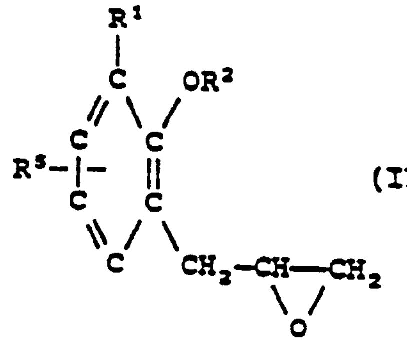

(II)

in which the radicals $R^1$, $R^2$ and $R^5$ have the meaning mentioned in respect of formula I, with a compound of the formula $R^4XH$, where $R^4$ has the meaning mentioned in respect of formula I, and X is O or S, to give a compound of the formula I in which $R^1$, $R^2$, $R^4$ and $R^5$ have the indicated meaning, $R^3$ is the hydroxyl group, and X denotes sulfur or oxygen, or B) reacting a compound of the formula III

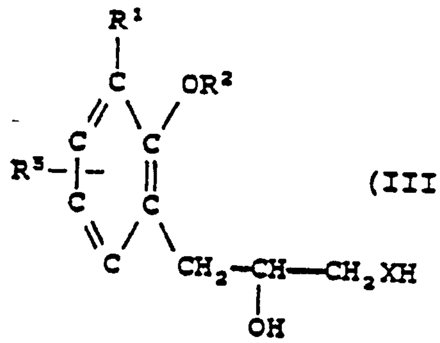

(III)

in which the radicals $R^1$, $R^2$ and $R^5$ have the meaning mentioned in respect of formula I, and X denotes O or S, with a compound of the formula $R^4Y$, where $R^4$ has the meaning mentioned in respect of formula I, and Y is a leaving group to give a compound of the formula I in which $R^1$, $R^2$, $R^4$ and $R^5$ have the indicated meaning, $R^3$ is the hydroxyl group and X denotes sulfur or oxygen, and, where appropriate, transforming a resulting compound by conversion into other compounds of the formula I.

2. The process as claimed in claim 1, in which is prepared a compound of the formula I, in which the radicals have the following meaning :

X is O, S, SO or $SO_2$ ;

$R^1$ is H, $C_1$-$C_4$-alkyl, $C_3$-$C_8$-cycloalkyl or $C_3$-$C_8$-cycloalkenyl ;

$R^2$ is H, $C_1$-$C_{10}$-alkyl (straight-chain or branched), $C_3$-$C_8$-cycloalkyl, phenyl-$C_1$-$C_{10}$-alkyl or phenoxy-$C_1$-$C_{10}$-alkyl, it being possible for the phenyl rings also to be substituted by 1-3 $C_1$-$C_4$-alkyl, $C_2$-$C_4$-alkenyl, $C_1$-$C_4$-alkoxy; $C_1$-$C_4$-alkanoyl, $C_1$-$C_4$-alkoxycarbonyl, hydroxyl or halogen radicals, or $R^2$ is pyridylmethyl or thienylmethyl ;

$R^3$ is OH ;

$R^4$ is $C_1$-$C_4$-alkyl, $C_2$-$C_4$-hydroxyalkyl, $C_3$-$C_4$-dihydroxyalkyl, $C_1$-$C_4$-alkoxy-$C_1$-$C_4$-alkyl, $C_1$-$C_4$-alkanoyloxy-$C_1$-$C_4$-alkyl, phenyl or phenyl-$C_1$-$C_4$-alkyl, it being possible for the phenyl rings to be substituted once or twice by HO, halogen, $C_1$-$C_4$-alkoxy or $C_1$-$C_4$-alkylthio, or $R^4$, is a group of the general formula $(CH_2)_nCOR^8$ or $(CH_2)_nR^9$ ;

$R^5$ is H or halogen ;

$R^7$ is $C_1$-$C_4$-alkyl ;

$R^8$ is OH, $C_1$-$C_4$-alkoxy ; $CH_2OH$, $OCH_2CO_2H$, $OCH_2CO_2R^7$, $OCH_2Ph$, NHOH, $NH_2$ ; $NHR^7$, $NR^7_2$, piperidino, pyrrolidino, morpholino, or phenoxy, it being possible for the phenoxy radical also to be substituted by carboxyl, $C_1$-$C_4$-alkoxycarbonyl, OH or $OCH_3$ ;

$R^9$ is tetrazol-5-yl ;

n is 1, 2 or 3.

3. The process as claimed in claim 1, in which is prepared a compound and of the formula I, in which the radicals have the following meaning :

X is S ;

$R^1$ is H, $CH_3$ or cyclopentyl ;

$R^2$ is H, benzyl, it being possible for the benzyl radical also to be substituted once or twice on the phenyl ring by methoxy or chlorine, is phenoxy-$C_2$-$C_4$-alkyl, it being possible for the phenyl ring also to be substituted by 1-3 $C_1$-$C_4$-alkyl, $C_1$-$C_4$-alkanoyl or hydroxy groups, or is pyridylmethyl or thienylmethyl ;

$R^3$ is OH ;

$R^4$ is $C_1$-$C_4$-alkyl, $C_2$-$C_4$-hydroxyalkyl, $C_3$-$C_4$-dihydroxyalkyl, $C_1$-$C_4$-alkoxy-$C_1$-$C_4$-alkyl, $C_1$-$C_4$-alkanoyloxy-$C_1$-$C_4$-alkyl, phenyl or benzyl, it being possible for the phenyl rings to be substituted once or twice by HO, methoxy or methylthio, or $R^4$ is a group of the general formula $(CH_2)_nCOR^8$ or $(CH_2)_nR^9$ ;

$R^5$ is H or chlorine ;

$R^7$ is $C_1$-$C_4$-alkyl ;

$R^8$ is OH, $C_1$-$C_4$-alkoxy, $CH_2OH$, $OCH_2CO_2H$, $OCH_2CO_2R^7$, $OCH_2Ph$, NHOH, $NH_2$, piperidino, pyrrolidino, morpholino, or phenoxy, it being possible for the phenoxy radical also to be substituted by carboxyl, $C_1$-$C_4$-alkoxycarbonyl, OH or $OCH_3$ ;

$R^9$ is tetrazol-5-yl ;

n is 1, 2 or 3.

4. The process as claimed in claim 1, wherein methyl (6RS)-7-(2-benzyloxyphenyl)-6-hydroxy-4-thiaheptanoate is prepared.

5. The process as claimed in claim 1, wherein methyl (6RS)-7-(2-benzyloxy-3-cyclopentylphenyl)-6-hydroxy-4-thiaheptanoate is prepared.

6. The process as claimed in claim 1, wherein methyl (6RS)-7-(2-benzyloxy-3-methylphenyl)-6-hydroxy-4-thiaheptanoate is prepared.

7. The process as claimed in claim 1, wherein methyl (6RS)-7-(2-benzyloxy-3,5-dimethylphenyl)-6-hydroxy-4-thiaheptanoate is prepared.

8. The process as claimed in claim 1, wherein (6RS)-7-(2-benzyloxy-3-cyclopentylphenyl)-6-hydroxy-4-thiaheptanoic acid is prepared.

9. The process as claimed in claim 1, wherein methyl (6RS)-7-(2-benzyloxy-5-chlorophenyl)-6-hydroxy-4-thiaheptanoate is prepared.

10. The process as claimed in claim 1, wherein (5RS)-6-(2-benzyloxy-3-cyclopentylphenyl)-3-thiahexane-1,5-diol is prepared.

11. A process for the preparation of a pharmaceutical composition, which comprises converting one or more of the compounds obtainable as claimed in claim 1 into a suitable dosage form.

**Revendications**

**Revendications pour les Etats contractants suivants : AT, BE, CH, DE, FR, GB, IT, LI, LU, NL, SE**

1. Composés de formule générale I

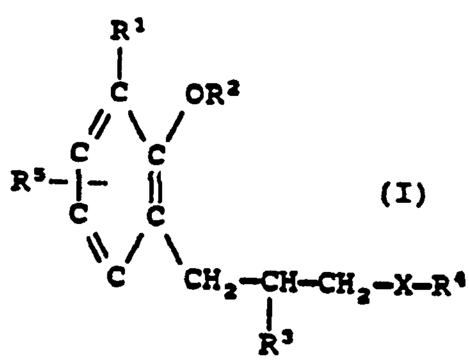

(I)

dans laquelle les radicaux ont les significations suivantes :

X est O, S, SO ou $SO_2$ ;

$R^1$ est H, un groupe alkyle en $C_1$-$C_{12}$, alcényle en $C_2$-$C_{12}$ ou alcynyle en $C_2$-$C_{12}$(à chaînes droites ou ramifiées), cycloalkyle en $C_3$-$C_8$, cycloalcényle en $C_3$-$C_8$, un atome d'halogène, un groupe phénoxy, $CF_3$, $NO_2$, OH, $OR^6$, COOH, $COOR^6$, CHO ou $COR^7$ ;

$R^2$ est H, un groupe alkyle en $C_1$-$C_{12}$, alcényle en $C_2$-$C_{12}$ ou alcynyle en $C_2$-$C_{12}$ (à chaînes droites ou rami-

fiées), cycloalkyle en $C_3$-$C_8$, cycloalcényle en $C_3$-$C_8$, phénylalkyle($C_1$-$C_{10}$), phénoxy-alkyle($C_1$-$C_{10}$), phénylalcényle-($C_2$-$C_{10}$)ou phényl-alcynyle($C_2$-$C_{10}$), les noyaux phényle pouvant être encore substitués par 1-3 atomes d'halogène ou radicaux alkyle en $C_1$-$C_4$, alcényle en $C_2$-$C_4$, alcoxy en $C_1$-$C_4$, alcanoyle en $C_1$-$C_4$, alcoxy($C_1$-$C_4$)-carbonyle ou hydroxy, ou $R^2$ est le groupe pyridylméthyle ou thiénylméthyle ;

$R^3$ est OH, $OR^6$, $OCOR^7$ ;

$R^4$ est un groupe alkyle en $C_1$-$C_4$, hydroxyalkyle en $C_2$-$C_4$, dihydroxyalkyle en $C_3$-$C_4$, alcoxy($C_1$-$C_4$)-alkyle($C_1$-$C_4$), alcanoyloxy($C_1$-$C_4$)-alkyle($C_1$-$C_4$), phényle ou phénylalkyle($C_1$-$C_4$), les noyaux phényle pouvant être une ou deux fois substitués par un ou des atomes d'halogène ou radicaux OH, alcoxy en $C_1$-$C_4$ ou alkyl($C_1$-$C_4$)-thio, ou $R^4$ est un groupe de formule générale $(CH_2)_nCOR^8$ ou $(CH_2)_nR^9$ ;

$R^5$ est un atome d'hydrogène ou d'halogène, $CF_3$, OH, un groupe alkyle en $C_1$-$C_4$ ou alcoxy en $C_1$-$C_4$ ;

$R^6$ est un groupe alkyle en $C_1$-$C_4$, allyle ou benzyle ;

$R^7$ est un groupe alkyle en $C_1$-$C_4$ ou phényle ;

$R^8$ est OH, un groupe alcoxy en $C_1$-$C_4$, $CH_2OH$, $OCH_2CO_2H$, $OCH_2CO_2R^7$, $OCH_2Ph$, NHOH, $NH_2$, $NHR^7$, $NR^7{}_2$, pipéridino, pyrrolidino, morpholino ou phénoxy, le reste phénoxy pouvant être encore substitué par un radical carboxy, alcoxy-($C_1$-$C_4$)-carbonyle, OH ou $OCH_3$ ;

$R^9$ est le groupe tétrazol-5-yle ou 2-oxo-1,3-dioxolanne-4-yle ;

n est 0, 1, 2, ou 3 ;

ainsi que les sels physiologiquement acceptables des composés de formule générale I, dans lesquels l'un des radicaux contient le groupe carboxy (COOH).

2. Composés selon la revendication 1, de formule générale I, dans laquelle les radicaux ont les significations suivantes :

X est O, S, SO ou $SO_2$ ;

$R^1$ est H ou un groupe alkyle en $C_1$-$C_4$, cycloalkyle en $C_3$-$C_8$ ou cycloalcényle en $C_3$-$C_8$ ;

$R^2$ est H ou un groupe alkyle en $C_1$-$C_{10}$ (à chaîne droite ou ramifiée) ; cycloalkyle en $C_3$-$C_8$, phényl-alkyle($C_1$-$C_{10}$) ou phénoxy-alkyle($C_1$-$C_{10}$), les noyaux phényle pouvant être encore substitués par 1-3 atomes d'halogène ou radicaux alkyle en $C_1$-$C_4$, alcényle en $C_2$-$C_4$, alcoxy en $C_1$-$C_4$, alcanoyle en $C_1$-$C_4$, alcoxy($C_1$-$C_4$)-carbonyle ou hydroxy, ou $R^2$ est le groupe pyridylméthyle ou thiénylméthyle ;

$R^3$ est OH ;

$R^4$ est un groupe alkyle en $C_1$-$C_4$, hydroxyalkyle en $C_2$-$C_4$, dihydroxyalkyle en $C_3$-$C_4$, alcoxy($C_1$-$C_4$)-alkyle($C_1$-$C_4$), alcanoyloxy($C_1$-$C_4$)-alkyle($C_1$-$C_4$), phényle ou phénylalkyle($C_1$-$C_4$), les noyaux phényle pouvant être une ou deux fois substitués par un ou des atomes d'halogène ou radicaux OH, alcoxy en $C_1$-$C_4$ ou alkyl($C_1$-$C_4$)-thio, ou $R^4$ est un groupe de formule générale $(CH_2)_n$ $COR^8$ ou $(CH_2)_nR^9$ ;

$R^5$ est un atome d'hydrogène ou d'halogène ;

$R^7$ est un groupe alkyle en $C_1$-$C_4$ ;

$R^8$ est un groupe OH, alcoxy en $C_1$-$C_4$, $CH_2OH$, $OCH_2CO_2H$, $OCH_2CO_2R^7$, $OCH_2Ph$, NHOH, $NH_2$, $NHR^7$, $NR^7{}_2$, pipéridino, pyrrolidino, morpholino ou phénoxy, le reste phénoxy pouvant être encore substitué par un radical carboxy, alcoxy-($C_1$-$C_4$)-carbonyle, OH ou $OCH_3$ ;

$R^9$ est le groupe tétrazol-5-yle ;

n est 1, 2 ou 3.

3. Composés selon la revendication 1, de formule générale I, dans laquelle les radicaux ont les significations suivantes :

X est S ;

$R^1$ est H ou le groupe $CH_3$ ou cyclopentyle ;

$R^2$ est H ou le groupe benzyle, le groupe benzyle pouvant être encore une ou deux fois substitué sur le noyau phényle par un ou des atomes de chlore ou par le radical méthoxy, un groupe phénoxy-alkyle($C_2$-$C_4$), le noyau phényle pouvant être encore substitué par 1-3 radicaux alkyle en $C_1$-$C_4$, alcanoyle en $C_1$-$C_4$ ou hydroxy, le groupe pyridylméthyle ou thiénylméthyle ;

$R^3$ est OH ;

$R^4$ est un groupe alkyle en $C_1$-$C_4$, hydroxyalkyle en $C_2$-$C_4$, dihydroxyalkyle en $C_3$-$C_4$, alcoxy($C_1$-$C_4$)-alkyle($C_1$-$C_4$), alcanoyloxy($C_1$-$C_4$)-alkyle($C_1$-$C_4$), phényle ou benzyle, les noyaux phényle pouvant être une ou deux fois substitués par le groupe OH, méthoxy ou méthylthio, ou $R^4$ est un groupe de formule générale $(CH_2)_nCOR^8$ ou $(CH_2)_nR^9$ ;

$R^5$ est un atome d'hydrogène ou de chlore ;

$R^7$ est un groupe alkyle en $C_1$-$C_4$ ;

$R^8$ est un groupe OH, alcoxy en $C_1$-$C_4$, $CH_2OH$, $OCH_2CO_2H$, $OCH_2CO_2R^7$, $OCH_2Ph$, NHOH, $NH_2$, pipéridino, pyrrolidino, morpholino ou phénoxy, le reste phénoxy pouvant être encore substitué par un radical carboxy, alcoxy($C_1$-$C_4$)-carbonyle, OH ou $OCH_3$ ;

$R^9$ est le groupe tétrazol-5-yle ;

n est 1, 2 ou 3.

4. (6RS)-7-(2-benzyloxyphényl)-6-hydroxy-4-thiaheptanoate de méthyle

5. (6RS)-7-(2-benzyloxy-3-cyclopentylphényl)-6-hydroxy-4-thiaheptanoate de méthyle

6. (6RS)-7-(2-benzyloxy-3-méthylphényl)-6-hydroxy-4-thiaheptanoate de méthyle

7. (6RS)-7-(2-benzyloxy-3,5-diméthylphényl)-6-hydroxy-4-thiaheptanoate de méthyle

8. Acide (6RS)-7-(2-benzyloxy-3-cyclopentylphényl)-6-hydroxy-4-thiaheptanoïque

9. (6RS)-7-(2-benzyloxy-5-chlorophényl)-6-hydroxy-4-thiaheptanoate de méthyle

10. (5RS)-6-(2-benzyloxy-3-cyclopentylphényl)-3-thiahexane-1,5-diol.

11. Procédé pour la préparation des composés selon la revendication 1, caractérisé en ce que A) on fait réagir un composé de formule générale II

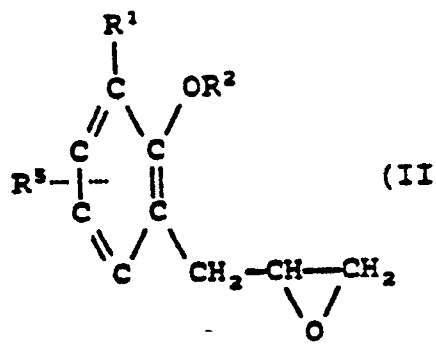

(II)

(II)

dans laquelle les radicaux $R^1$, $R^2$ et $R^5$ ont les significations données dans la revendication 1, avec un composé de formule générale $R^4XH$, $R^4$ ayant la signification donnée dans la revendication 1 et X étant O ou S, pour aboutir à un composé de formule I dans lequel $R^1$, $R^2$, $R^4$ et $R^5$ ont les significations indiquées, $R^3$ est le groupe hydroxy et X représente un atome de soufre ou d'oxygène, ou B) on fait réagir un composé de formule générale III

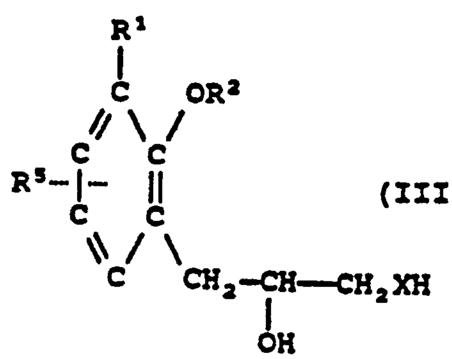

(III)

(III)

dans laquelle les radicaux $R^1$, $R^2$ et $R^5$ ont les significations données dans la revendication 1, et X représente O ou S, avec un composé de formule générale $R^4Y$, $R^4$ ayant la signification donnée dans la revendication 1 et Y étant un groupe séparable, pour aboutir à un composé de formule I dans lequel $R^1$, $R^2$, $R^4$ et $R^5$ ont les significations indiquées, $R^3$ est le groupe hydroxy et X représente un atome de soufre ou d'oxygène, et éventuellement on transforme un composé obtenu par conversion en d'autres composés de formule I.

12. Compositions pharmaceutiques contenant un ou plusieurs des composés selon la revendication 1.

13. Composé selon la revendication 1, pour le traitement de maladies qui sont caractérisées par un taux élevé de leucotriènes, telles que l'asthme, les réactions cutanées allergiques, le psoriasis, la colite ulcéreuse, la polyarthrite rhumatoïde et l'état de choc.

14. Composition pharmaceutique selon la revendication 12, pour le traitement de maladies qui sont caractérisées par un taux élevé de leucotriènes, telles que l'asthme, les réactions cutanées allergiques, le psoriasis, la colite ulcéreuse, la polyarthrite rhumatoïde et l'état de choc.

**Revendications pour les Etats contractants suivants : ES, GR**

1. Procédé pour la préparation de composés de formule générale I

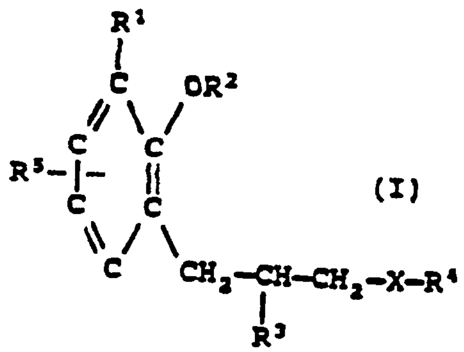

(I) (I)

dans laquelle les radicaux ont les significations suivantes :

X est O, S, SO ou $SO_2$ ;

$R^1$ est H, un groupe alkyle en $C_1$-$C_{12}$, alcényle en $C_2$-$C_{12}$ ou alcynyle en $C_2$-$C_{12}$(à chaînes droites ou ramifiées), cycloalkyle en $C_3$-$C_8$, cycloalcényle en $C_3$-$C_8$, un atome d'halogène, un groupe phénoxy, $CF_3$, $NO_2$, OH, $OR^6$, COOH, $COOR^6$, CHO ou $COR^7$ ;

$R^2$ est H, un groupe alkyle en $C_1$-$C_{12}$, alcényle en $C_2$-$C_{12}$ ou alcényle en $C_2$-$C_{12}$ (à chaînes droites ou ramifiées), cycloalkyle en $C_3$-$C_8$, cycloalcényle en $C_3$-$C_8$, phénylalkyle($C_1$-$C_{10}$), phénoxy-alkyle($C_1$-$C_{10}$), phénylalcényle-($C_2$-$C_{10}$) ou phényl-alcynyle($C_2$-$C_{10}$), les noyaux phényle pouvant être encore substitués par 1-3 atomes d'halogène ou radicaux alkyle en $C_1$-$C_4$, alcényle en $C_2$-$C_4$, alcoxy en $C_1$-$C_4$, alcanoyle en $C_1$-$C_4$, alcoxy($C_1$-$C_4$)-carbonyle ou hydroxy, ou $R^2$ est le groupe pyridylméthyle ou thiénylméthyle ;

$R^3$ est OH, $OR^6$, $OCOR^7$ ;

$R^4$ est un groupe alkyle en $C_1$-$C_4$, hydroxyalkyle en $C_2$-$C_4$, dihydroxyalkyle en $C_3$-$C_4$, alcoxy($C_1$-$C_4$)-alkyle($C_1$-$C_4$), alcanoyloxy($C_1$-$C_4$)-alkyle($C_1$-$C_4$), phényle ou phénylalkyle($C_1$-$C_4$), les noyaux phényle pouvant être une ou deux fois substitués par un ou des atomes d'halogène ou radicaux OH, alcoxy en $C_1$-$C_4$ ou alkyl($C_1$-$C_4$)-thio, ou $R^4$ est un groupe de formule générale $(CH_2)_nCOR^8$ ou $(CH_2)_nR^9$ ;

$R^5$ est un atome d'hydrogène ou d'halogène, $CF_3$, OH, un groupe alkyle en $C_1$-$C_4$ ou alcoxy en $C_1$-$C_4$ ;

$R^6$ est un groupe alkyle en $C_1$-$C_4$, allyle ou benzyle ;

$R^7$ est un groupe alkyle en $C_1$-$C_4$ ou phényle ;

$R^8$ est OH, un groupe alcoxy en $C_1$-$C_4$, $CH_2OH$, $OCH_2CO_2H$, $OCH_2 CO_2 R^7$, $OCH_2$ Ph, NHOH, $NH_2$, $NHR^7$, $NR^7_2$, pipéridino, pyrrolidino, morpholino ou phénoxy, le reste phénoxy pouvant être encore substitué par un radical carboxy, alcoxy-($C_1$-$C_4$)-carbonyle, OH ou $OCH_3$ ;

$R^9$ est le groupe tétrazol-5-yle ou 2-oxo-1,3-dioxolanne-4-yle ;

n est 0, 1, 2 ou 3 ;

ainsi que des sels physiologiquement acceptables des composés de formule générale I, dans lesquels l'un des radicaux contient un groupe carboxy (COOH), caractérisé en ce que A) on fait réagir un composé de formule générale II

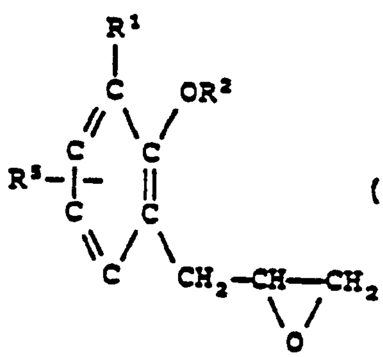

(II) (II)

dans laquelle les radicaux $R^1$, $R^2$ et $R^5$ ont les significations données à propos de la formule I, avec un composé

de formule générale $R^4XH$, $R^4$ ayant la signification donnée à propos de la formule I et X étant O ou S, pour aboutir à un composé de formule I dans $R^1$, $R^2$, $R^4$ et $R^5$ ont les significations indiquées, $R^3$ est le groupe hydroxy et X représente un atome de soufre ou d'oxygène, ou B) on fait réagir un composé de formule générale III

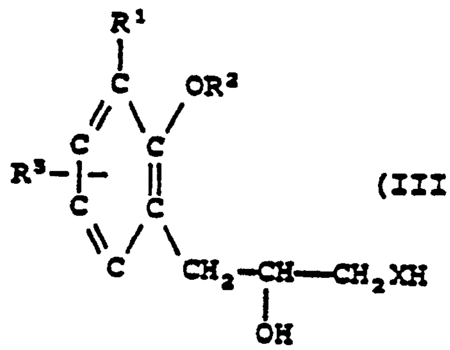

(III) (III)

dans laquelle les radicaux $R^1$, $R^2$ et $R^5$ ont les significations données à propos de la formule I, et X représente O ou S, avec un composé de formule générale $R^4Y$, $R^4$ ayant la signification donnée à propos de la formule I et Y étant un groupe séparable, pour aboutir à un composé de formule I dans lequel $R^1$, $R^2$, $R^4$ et $R^5$ ont les significations indiquées, $R^3$ est le groupe hydroxy et X représente un atome de soufre ou d'oxygène, et éventuellement on transforme un composé obtenu par conversion en d'autres composés de formule I.

2. Procédé selon la revendication 1, caractérisé en ce que l'on prépare un composé de formule générale I, dans laquelle les radicaux ont les significations suivantes :

X est O, S, SO ou $SO_2$ ;

$R^1$ est H ou un groupe alkyle en $C_1$-$C_4$, cycloalkyle en $C_3$-$C_8$ ou cycloalcényle en $C_3$-$C_8$ ;

$R^2$ est H ou un groupe alkyle en $C_1$-$C_{10}$ (à chaîne droite ou ramifiée) ; cycloalkyle en $C_3$-$C_8$, phényl-alkyle($C_1$-$C_{10}$) ou phénoxy-alkyle($C_1$-$C_{10}$), les noyaux phényle pouvant être encore substitués par 1-3 atomes d'halogène ou radicaux alkyle en $C_1$-$C_4$, alcényle en $C_2$-$C_4$, alcoxy en $C_1$-$C_4$, alcanoyle en $C_1$-$C_4$, alcoxy($C_1$-$C_4$)-carbonyle ou hydroxy, ou $R^2$ est le groupe pyridylméthyle ou thiénylméthyle ;

$R^3$ est OH ;

$R^4$ est un groupe alkyle en $C_1$-$C_4$, hydroxyalkyle en $C_2$-$C_4$, dihydroxyalkyle en $C_3$-$C_4$, alcoxy($C_1$-$C_4$)-alkyle($C_1$-$C_4$), alcanoyloxy($C_1$-$C_4$)-alkyle($C_1$-$C_4$), phényle ou phénylalkyle($C_1$-$C_4$), les noyaux phényle pouvant être une ou deux fois substitués par un ou des atomes d'halogène ou radicaux OH, alcoxy en $C_1$-$C_4$ ou alkyl($C_1$-$C_4$)-thio, ou $R^4$ est un groupe de formule générale $(CH_2)_nCOR^8$ ou $(CH_2)_nR^9$ ;

$R^5$ est un atome d'hydrogène ou d'halogène ;

$R^7$ est un groupe alkyle en $C_1$-$C_4$ ;

$R^8$ est un groupe OH, alcoxy en $C_1$-$C_4$, $CH_2OH$, $OCH_2CO_2H$, $OCH_2CO_2R^7$, $OCH_2Ph$, NHOH, $NH_2$, $NHR^7$, $NR^7{}_2$, pipéridino, pyrrolidino, morpholino ou phénoxy, le reste phénoxy pouvant être encore substitué par un radical carboxy, alcoxy-($C_1$-$C_4$)-carbonyle, OH ou $OCH_3$ ;

$R^9$ est le groupe tétrazol-5-yle ;

n est 1, 2 ou 3.

3. Procédé selon la revendication 1, caractérisé en ce que l'on prépare un composé de formule générale I, dans laquelle les radicaux ont les significations suivantes :

X est S ;

$R^1$ est H ou le groupe $CH_3$ ou cyclopentyle ;

$R^2$ est H ou le groupe benzyle, le groupe benzyle pouvant être encore une ou deux fois substitué sur le noyau phényle par un ou des atomes de chlore ou par le radical méthoxy, un groupe phénoxy-alkyle($C_2$-$C_4$), le noyau phényle pouvant être encore substitué par 1-3 radicaux alkyle en $C_1$-$C_4$, alcanoyle en $C_1$-$C_4$ ou hydroxy, le groupe pyridylméthyle ou thiénylméthyle ;

$R^3$ est OH ;

$R^4$ est un groupe alkyle en $C_1$-$C_4$, hydroxyalkyle en $C_2$-$C_4$, dihydroxyalkyle en $C_3$-$C_4$, alcoxy($C_1$-$C_4$)-alkyle($C_1$-$C_4$), alcanoyloxy($C_1$-$C_4$)-alkyle($C_1$-$C_4$), phényle ou benzyle, les noyaux phényle pouvant être une ou deux fois substitués par le groupe OH, méthoxy ou méthylthio, ou $R^4$ est un groupe de formule générale $(CH_2)_nCOR^8$ ou $(CH_2)_nR^9$ ;

$R^5$ est un atome d'hydrogène ou de chlore ;

$R^7$ est un groupe alkyle en $C_1$-$C_4$ ;

$R^8$ est un groupe OH, alcoxy en $C_1$-$C_4$, $CH_2OH$, $OCH_2CO_2H$, $OCH_2CO_2R^7$, $OCH_2Ph$, NHOH, $NH_2$, pipéri-

dino, pyrrolidino, morpholino ou phénoxy, le reste phénoxy pouvant être encore substitué par un radical carboxy, alcoxy($C_1$-$C_4$)-carbonyle, OH ou $OCH_3$ ;

$R^9$ est le groupe tétrazol-5-yle ;

n est 1, 2 ou 3.

4. Procédé selon la revendication 1, caractérisé en ce que l'on prépare le (6RS)-7-(2-benzyloxyphényl)-6-hydroxy-4-thiaheptanoate de méthyle

5. Procédé selon la revendication 1, caractérisé en ce que l'on prépare le (6RS)-7-(2-benzyloxy-3-cyclopentylphényl-6-hydroxy-4-thiaheptanoate de méthyle

6. Procédé selon la revendication 1, caractérisé en ce que l'on prépare le (6RS)-7-(2-benzyloxy-3-méthylphényl)-6-hydroxy-4-thiaheptanoate de méthyle

7. Procédé selon la revendication 1, caractérisé en ce que l'on prépare le (6RS)-7-(2-benzyloxy-3,5-diméthylphényl)6-hydroxy-4-thiaheptanoate de méthyle

8. Procédé selon la revendication 1, caractérisé en ce que l'on prépare l'acide (6RS)-7-(2-benzyloxy-3-cyclopentylphényl)-6-hydroxy-4-thiaheptanoïque

9. Procédé selon la revendication 1, caractérisé en ce que l'on prépare le (6RS)-7-(2-benzyloxy-5-chlorophényl)-6-hydroxy-4-thiaheptanoate de méthyle

10. Procédé selon la revendication 1, caractérisé en ce que l'on prépare le (5RS)-6-(2-benzyloxy-3-cyclopentylphényl)-3-thiahexane-1,5-diol.

11. Procédé pour la préparation d'une composition pharmaceutique, caractérisé en ce que l'on met sous une forme pharmaceutique appropriée un ou plusieurs des composés préparables selon la revendication 1.